# EUROPEAN PATENT SPECIFICATION

(11) **EP 4 387 690 B1**
(45) Date of publication and mention of the grant of the patent: **18.11.2026**
(21) Application number: 22769820.6
(22) Date of filing: 19.08.2022
(51) Int. Cl.: A61M 5/315, A61M 5/32, A61M 5/50, A61M 5/20, A61M 5/24

(54) **THERAPEUTIC AGENT DELIVERY DEVICE WITH USER INPUT**
VORRICHTUNG ZUR ABGABE EINES THERAPEUTISCHEN MITTELS MIT BENUTZEREINGABE
DISPOSITIF D'ADMINISTRATION D'AGENT THÉRAPEUTIQUE AVEC ENTRÉE UTILISATEUR

(30) Priority: 20.08.2021 US 202163260453 P
(43) Date of publication of application: 26.06.2024
(73) Proprietor: Eli Lilly and Company, Indianapolis, IN 46285 (US)
(72) Inventor: HOLLEY, David Arthur, Indianapolis, Indiana 46206-6288 (US); MARSHALL, Michael Lewis, Indianapolis, Indiana 46206-6288 (US)
(74) Representative: J A Kemp LLP
(86) International application number: PCT/US2022/040829
(87) International publication number: WO 2023/023292

(56) References cited:
- WO-A1-2020/131552
- US-A1- 2012 323 177
- US-A1- 2013 324 925
- US-A1- 2015 045 729
- US-A1- 2020 297 933

## Description

### BACKGROUND

The present disclosure pertains to therapeutic agent delivery devices, and, in particular, to a portable therapeutic agent delivery device such as an injector pen.

Patients suffering from a number of different diseases frequently must inject themselves with medication. To allow a person to conveniently and accurately self-administer medicine, a variety of devices broadly known as injector pens or injection pens have been developed. Generally, these pens are equipped with a cartridge including a piston and one or more doses of liquid medication. A drive member, extending from within a base of the injector pen and operably connected with typically more rearward mechanisms of the pen that control drive member motion, is movable forward to advance the piston in the cartridge in such a manner to dispense the contained medication from an outlet at the opposite cartridge end, typically through a needle that penetrates a stopper at that opposite end. In disposable pens, after a pen has been used and exhausted the supply of medication within the cartridge, the entire pen is discarded by a user, who may then begin using a replacement pen. In reusable pens, after a pen has been used and exhausted the supply of medication within the cartridge, the pen is disassembled, the spent cartridge is replaced with a fresh cartridge, and the pen is reassembled for its subsequent use.

It would be desirable to provide a therapeutic agent delivery device with improved features, such as a providing a reusable device that facilitates ease of dispensing medication and/or ease of replacement of a spent cartridge with a fresh cartridge.

US 2015/045729 A1 discloses an autoinjector apparatus which comprises a single-use cassette and an autoinjector.

US 2020/297933 A1 discloses auto-injectors and associated assemblies and methods for delivery of liquid medicament in a controlled manner.

WO 2020/131552 A1 discloses a therapeutic agent delivery system comprising a housing. A therapeutic agent delivery assembly is carried by the housing. The therapeutic agent delivery assembly comprises a chamber having a passageway. A therapeutic agent is carried in the passageway, and a needle is in communication with the passageway. The therapeutic agent delivery assembly may be translatable relative to the housing from a stowed configuration to a deployed configuration. Actuation of a user input may translate the therapeutic agent delivery assembly from the stowed configuration to the deployed configuration. An input restraint may be rotatable relative to the housing from a first rotational configuration to a second rotational configuration. In the first rotational configuration the input restraint may inhibit actuation of the user input, and in the second rotational configuration the input restraint may permit actuation of the user input.

US 2013/324925A1 discloses an auto-injector for administering a dose of a liquid medicament, comprising: an elongate tubular case, a carrier subassembly comprising a tubular carrier slidably arranged inside the case, the carrier adapted to contain a syringe with a hollow injection needle, a drive spring and a plunger for forwarding load of the drive spring to a stopper of the syringe, wherein the syringe is lockable for joint axial translation with the carrier, wherein the drive spring acts between the carrier and the plunger, wherein the plunger in an initial state is locked to the carrier by a ramped engagement to the carrier and by being prevented from rotating out of this ramped engagement depending on a relative longitudinal position of the carrier in the case, wherein the plunger is arranged to be released for rotating out of the ramped engagement upon the needle reaching at least nearly a predefined injection depth on translation of the carrier in the proximal direction for advancing the needle beyond a proximal end of the auto-injector.

US 2012/323177 A1 discloses an automatic injection apparatus including a delay mechanism for properly delivering medication prior to the needled syringe of the apparatus being retracted. In one form, the delay mechanism includes a shuttle (170) for the syringe, a follower (250), a locking member, a damping compound between the follower and a supporting surface (308) to dampen rotation of the follower relative to the shuttle, and a dual functioning biasing member (290) acting between the shuttle and the follower. When the locking member moves to a release position during an injection, the dual functioning biasing member first provides a torsional force to force the follower to rotate relative to the shuttle from a latching position to an unlatching position, and then the dual functioning biasing member provides an axial force to force the shuttle axially relative to the follower to move the shuttle for retracting the syringe needle into the housing of the apparatus after injection.

### SUMMARY

The scope of the invention is defined in the appended claims. Any "aspect", "example" and "embodiment" of the description not falling within the scope of the claims does not form part of the invention and is provided for illustrative purposes only.

According to an embodiment of the present disclosure, a therapeutic agent delivery device includes a disposable portion and a reusable portion. The disposable portion including a first housing comprising a distal end, and a syringe assembly carried by the first housing. The syringe assembly including a chamber comprising a passageway, and a needle in communication with the passageway. The syringe assembly is translatable relative to the first housing from a stowed configuration to a deployed configuration. In the stowed configuration, the needle is disposed proximally relative to the distal end of the first housing, and in the deployed configuration the needle at least partially extending distally from the distal end of the first housing. The reusable portion detachably carries the disposable portion. The reusable portion including a second housing, a drive mechanism carried by the second housing and coupled to the syringe assembly, and a user input. The user input is carried by the second housing and operatively coupled to the drive mechanism. In response to actuation of the user input, the drive mechanism is configured to translate the syringe assembly relative to the first housing from the stowed configuration and the deployed configuration. The user input includes a first configuration and a second configuration prior to actuation, and in the first configuration the drive mechanism is inhibiting actuation of the user input, and in the second configuration the drive mechanism is permitting actuation of the user input.

According to another embodiment, a method of moving a user input of a therapeutic agent delivery device is described. The delivery device includes a syringe assembly carried by the housing. The syringe assembly including a chamber comprising a passageway, and a needle in communication with the passageway. The syringe assembly is translatable relative to the housing from a stowed configuration to a deployed configuration, and in the stowed configuration the needle is disposed proximally relative to the distal end of the housing, and in the deployed configuration the needle at least partially extending distally from the distal end of the housing. A drive mechanism is carried by the housing and coupled to the syringe assembly. A user input carried by the housing and operatively coupled to the drive mechanism. In response to actuation, of the user input the drive mechanism is configured to translate the syringe assembly relative to the first housing from the stowed configuration and the deployed configuration. The user input includes a first configuration and a second configuration prior to actuation. In the first configuration, the drive mechanism is inhibiting actuation of the user input, and in the second configuration the drive mechanism is permitting actuation of the user input. The method includes the steps of: providing the therapeutic agent delivery device with the user input in the first configuration; detecting a presence of a needle shield, wherein the needle shield is detachably coupled to the distal end of the housing and obscuring the needle in the stowed configuration, the therapeutic agent delivery device inhibiting actuation of the user input when the sensor detects that the needle shield is coupled to the distal end of the first housing; and actuating the user input to the second configuration when the sensor detects that the needle shield is detached from the distal end of the housing.

### BRIEF DESCRIPTION OF THE DRAWINGS

The above-mentioned and other advantages and objects of this invention, and the manner of attaining them, will become more apparent, and the invention itself will be better understood, by reference to the following description of embodiments of the invention taken in conjunction with the accompanying drawings, wherein:
FIG. 1 is a top perspective view of a therapeutic agent delivery device according to an embodiment of the present disclosure.
FIG. 2 is a bottom perspective view of the therapeutic agent delivery device of FIG. 1; a disposable portion is shown detached from a reusable portion.
FIG. 3 is a transverse sectional view of the therapeutic agent delivery device along line 3-3 of FIG. 1; a syringe assembly is shown in a stowed configuration.
FIG. 4 is a transverse sectional view of a distal end of the therapeutic agent delivery device of FIG. 1; the syringe assembly is shown in a deployed configuration.
FIG. 5 is a schematic representation of an electronics assembly of the therapeutic agent delivery device of FIG. 1.
FIG. 6 is a detail transverse sectional view of a proximal end of the therapeutic agent delivery device within line 6 of FIG. 3.
FIG. 7 is a cross sectional view of the proximal end of the therapeutic agent delivery device along line 7-7 of FIG. 1.
FIG. 8 is a cross sectional view of the proximal end of the therapeutic agent delivery device along line 8-8 of FIG. 1.
FIG. 9 is a detail transverse sectional view of the proximal end of the therapeutic agent delivery device of FIG. 1 in a first configuration.
FIG. 10 is a cross sectional view of the proximal end of the therapeutic agent delivery device of FIG. 1 in the first configuration.
FIG. 11 is a detail transverse sectional view of the proximal end of the therapeutic agent delivery device of FIG. 1 in a second configuration.
FIG. 12 is a cross sectional view of the proximal end of the therapeutic agent delivery device of FIG. 1 in the second configuration.
FIG. 13 is a detail transverse sectional view of the proximal end of the therapeutic agent delivery device of FIG. 1 in a third configuration.
FIG. 14 is a cross sectional view of the proximal end of the therapeutic agent delivery device of FIG. 1 in the third configuration.
FIG. 15 is a detail transverse sectional view of the proximal end of the therapeutic agent delivery device of FIG. 1 in a fourth configuration.
FIG. 16 is a cross sectional view of the proximal end of the therapeutic agent delivery device of FIG. 1 in the fourth configuration.
FIG. 17 is a perspective view of a therapeutic agent delivery mechanism of the therapeutic agent delivery device of FIG. 1.
FIG. 18 is a detail transverse sectional view of the therapeutic agent delivery device within line 18 of FIG. 3.
FIG. 19 is a perspective transverse sectional view of a therapeutic agent delivery mechanism and a syringe assembly according to an embodiment of the present disclosure.
FIG. 20 is a transverse sectional view of the therapeutic agent delivery mechanism and the syringe assembly of FIG. 19; the therapeutic agent delivery mechanism is illustrated in a retracted configuration.
FIG. 21 is a transverse sectional view of the therapeutic agent delivery mechanism and the syringe assembly of FIG. 19; the therapeutic agent delivery mechanism is illustrated in an extended configuration.
FIG. 22 is a perspective view of a disposable portion of the therapeutic agent delivery device of FIG. 1.
FIG. 23 is a transverse sectional view of the disposable portion along line 23-23 of FIG. 22.
FIG. 24 is an exploded perspective view of the disposable portion of FIG. 22.
FIG. 25 is a perspective view of a disposable portion according to an embodiment of the present disclosure.
FIG. 26 is an exploded perspective view of the disposable portion of FIG. 25.
FIG. 27 is a side view of the disposable portion of FIG. 25 in a first configuration.
FIG. 28 is a side view of the disposable portion of FIG. 25 in a second configuration.
FIG. 29 is a side view of the disposable portion of FIG. 25 in a third configuration.
FIG. 30 is an exploded perspective view of a disposable portion according to another embodiment of the present disclosure.
FIG. 31 is a detail side view of the disposable portion of FIG. 30.
FIG. 32 is a perspective view of a securing mechanism of the therapeutic agent delivery device of FIG. 1.
FIG. 33 is a transverse sectional view of the securing mechanism along line 33- 33 of FIG. 32.
FIG. 34 is a side view of the securing mechanism of FIG. 32 and a disposable portion in a first configuration.
FIG. 35 is a side view of the securing mechanism of FIG. 32 and the disposable portion in a second configuration.
FIG. 36 is a side view of the securing mechanism of FIG. 32 and the disposable portion in a third configuration.
FIG. 37 is a side view of the securing mechanism of FIG. 32 and the disposable portion in a fourth configuration.
FIG. 38 is a side view of the securing mechanism of FIG. 32 and the disposable portion in a fifth configuration.
FIG. 39 is a side view of the securing mechanism of FIG. 32 and the disposable portion in a sixth configuration.
FIG. 40 is a transverse sectional view of a securing mechanism according to an embodiment of the present disclosure.
FIG. 41 is a side view of the securing mechanism of FIG. 40 and a disposable portion in a second configuration.
FIG. 42 is a side view of the securing mechanism of FIG. 40 and the disposable portion in a third configuration.
FIG. 43 is a side view of the securing mechanism of FIG. 40 and the disposable portion in a fourth configuration.
FIG. 44 is a side view of the securing mechanism of FIG. 40 and the disposable portion in a fifth configuration.
FIG. 45 is a side view of the securing mechanism of FIG. 40 and the disposable portion in a sixth configuration.
FIG. 46 is a side view of the securing mechanism of FIG. 40 and the disposable portion in a seventh configuration.

Corresponding reference characters indicate corresponding parts throughout the several views. Although the drawings represent embodiments of the present invention, the drawings are not necessarily to scale, and certain features may be exaggerated or omitted in some of the drawings in order to better illustrate and explain the present invention.

### DETAILED DESCRIPTION

Therapeutic agent delivery devices according to the present disclosure carry and dispense one or more therapeutic agents, which may also be referred to as medications or drugs. Such therapeutic agents may include, for example, epinephrine, anaesthetics, analgesics, steroids, insulins, insulin analogs such as insulin lispro or insulin glargine, insulin derivatives, GLP-1 receptor agonists such as dulaglutide or liraglutide, glucagon, glucagon analogs, glucagon derivatives, gastric inhibitory polypeptide (GIP), GIP analogs, GIP derivatives, combined GIP/GLP-1 agonists such as tirzepatide, basal insulins, oxyntomodulin analogs, oxyntomodulin derivatives, therapeutic antibodies including but not limited to IL-23 antibody analogs or derivatives, such as mirikizumab, IL-17 antibody analogs or derivatives, such as ixekizumab, therapeutic agents for pain-related treatments, such as galcanzeumab or lasmiditan, or lebrikizumab and any therapeutic agent that is capable of delivery by the devices described herein. Therapeutic agent delivery devices according to the present disclosure are operated in a manner generally as described herein by a user (for example, a healthcare professional, a caregiver, or another person) to deliver one or more therapeutic agents to a patient (for example, another person or the user).

Any directional references used with respect to any of the Figures, such as right or left, up or down, or top or bottom, are intended for convenience of description, and does not limit the present disclosure or any of its components to any particular positional or spatial orientation.

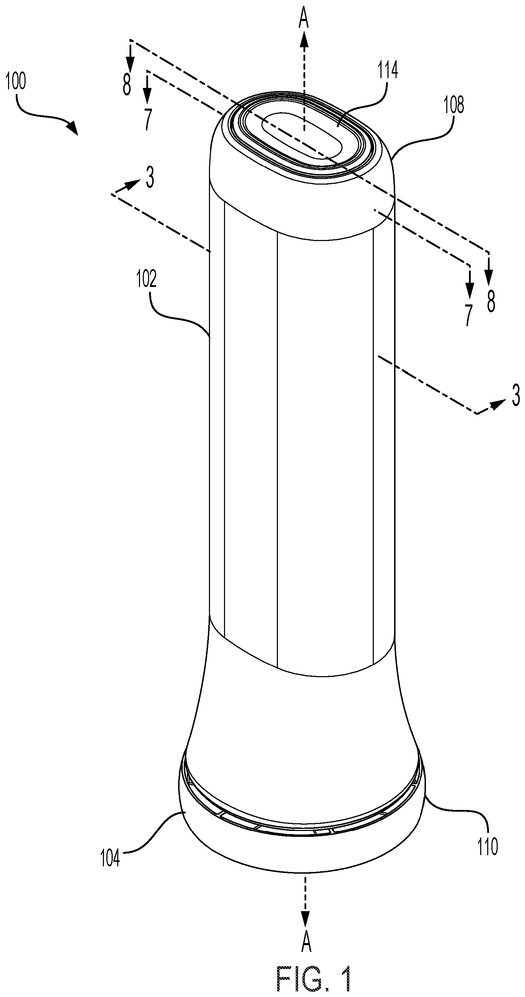

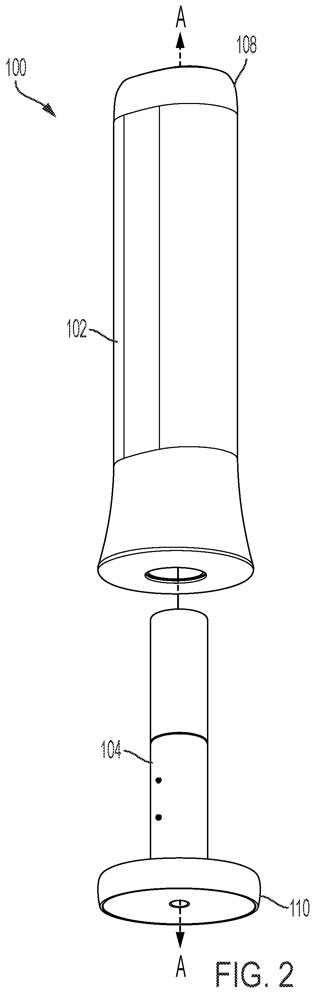

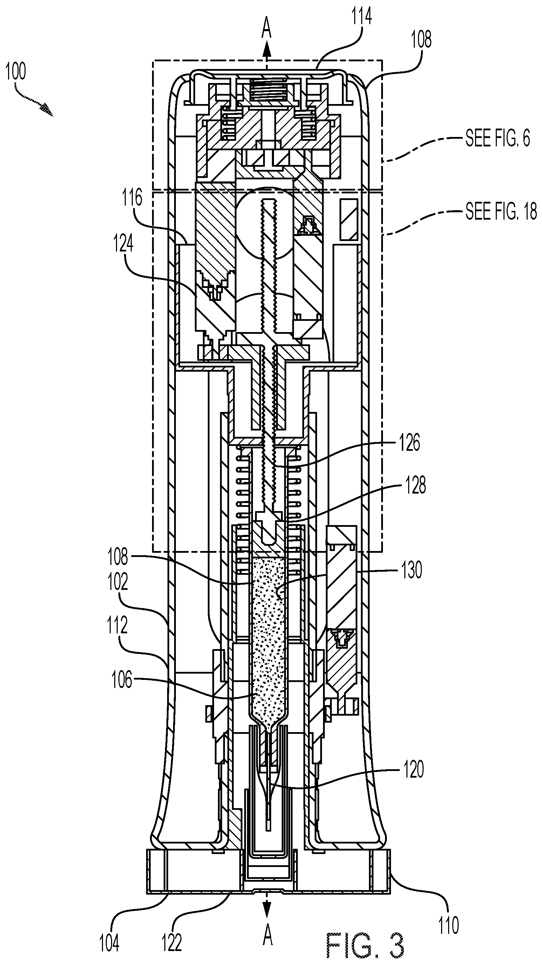

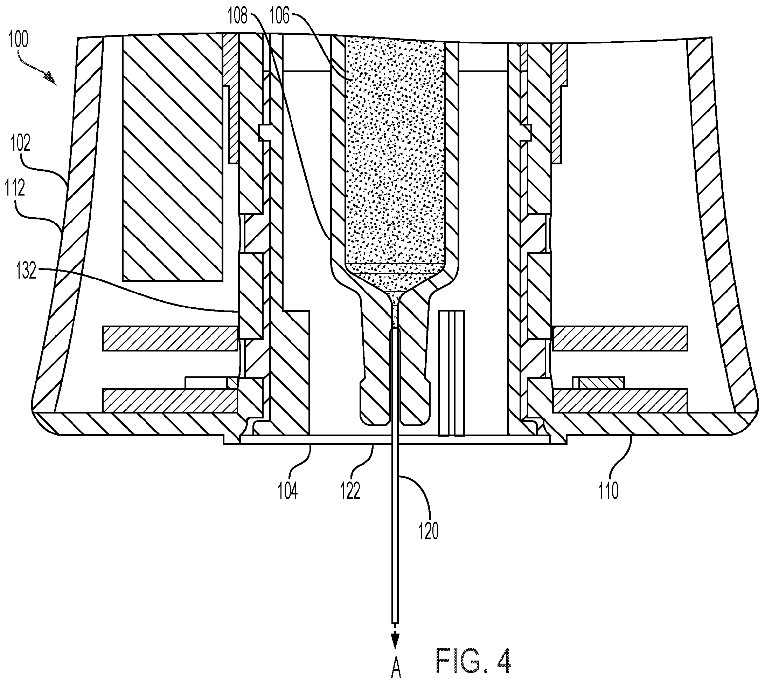

FIGS. 1-4 illustrate a therapeutic agent delivery device 100 according to an exemplary embodiment of the present disclosure. Illustratively, the therapeutic agent delivery device 100 has an injector pen-like shape, although other shapes may alternatively be used. The therapeutic agent delivery device 100 generally includes a reusable portion 102, which may also be referred to as a drive portion, and a disposable portion 104, which may also be referred to as a drug carrying portion or cartridge. The reusable portion 102 facilitates delivery of a therapeutic agent 106 (FIGS. 3 and 4) from the disposable portion 104. In addition, the disposable portion 104 detachably couples to the reusable portion 102 such that after the therapeutic agent 106 has been delivered from the disposable portion 104, the disposable portion 104 may be detached from the reusable portion 102 and discarded. Another disposable portion (not shown - for example, having the same or different features than the disposable portion 104) may then be attached to the reusable portion 102, and the therapeutic agent delivery device 100 is thereby ready for subsequent use.

The therapeutic agent delivery device 100 also includes a proximal end 108 and an opposite distal end 110. During use of the therapeutic agent delivery device 100, the proximal end 108 would be further from the patient and configured to be actuated by the user, and the distal end 110 would be closer to the patient and configured to deliver the therapeutic agent 106 to the patient. The therapeutic agent delivery device 100 also includes a longitudinal axis A extending between the proximal end 108 and the distal end 110. These and other features of the therapeutic agent delivery device 100 are described in further detail below.

With specific reference to the transverse sectional views of FIGS. 3 and 4, internal components and other features of the reusable portion 102 and the disposable portion 104 are illustrated. Generally, the reusable portion 102 includes a housing 112 that movably carries a user input 114 and a drive mechanism 116 (both shown in FIG. 3). The user input 114 is actuatable (for example, depressible) by a user to actuate the drive mechanism 116. The drive mechanism 116 thereby translates distally to drive a syringe assembly 118 of the disposable portion 104. More specifically, the drive mechanism 116 translatably drives the syringe assembly 118 from a stowed configuration (FIG. 3) to a deployed configuration (FIG. 4). In the stowed configuration, a needle 120 of the syringe assembly 118 is disposed proximally relative to a distal end 122 of the disposable portion 104. Stated another way, in the stowed configuration the needle 120 is retracted within the device 100. In the deployed configuration (FIG. 4), the needle 120 at least partially extends distally from the distal end 122 of the disposable portion 104. As a result, in the deployed configuration the needle 120 is configured to pierce the skin of a patient.

With specific reference to FIG. 3, the drive mechanism 116 of the reusable portion 102 also includes a plunger mechanism or therapeutic agent delivery mechanism 124. The therapeutic agent delivery mechanism 124 is actuatable to discharge the therapeutic agent 106 from the syringe assembly 118. More specifically, when the syringe assembly 118 is in the deployed configuration, the therapeutic agent delivery mechanism 124 is actuatable to distally translate a shaft or plunger 126 of the syringe assembly 118. The plunger 126 distally drives a piston 128 carried in a therapeutic agent-carrying passageway 130 of the syringe assembly 118, which causes the therapeutic agent 106 to be discharged via the needle 120. Additional details of these components are described below.

With brief specific reference to FIG. 4, the distal end 110 of the device 100 further includes a securing mechanism 132 for selectively securing the disposable portion 104 to the reusable portion 102. The securing mechanism 132 also facilitates easily attaching the disposable portion 104 to the reusable portion 102 and detaching the disposable portion 104 from the reusable portion 102. The components and features of the securing mechanism 132 are described in further detail below.

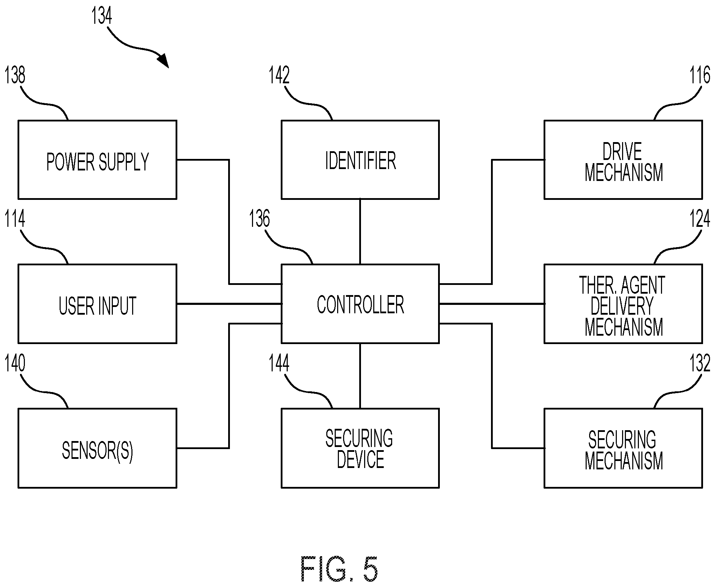

Referring to FIG. 5, in addition to the above components, the therapeutic agent delivery device 100 also includes an electronics assembly 134 that facilitates operating the device 100 in the manners described herein. The electronics assembly 134 includes an electronic controller 136 that is operatively coupled to and receives power from a power supply 138, such as a battery. The electronic controller 136 also operatively couples to the user input 114 and one or more sensors 140. As described in further detail below, the sensors 140 may sense, for example, actuation of components of the device 100, positions of components of the device 100 relative to each other, and/or the position of the device 100 relative to a patient. The controller 136 further operatively couples to the drive mechanism 116 (FIG. 3), the therapeutic agent delivery mechanism 124 (FIG. 3), and the securing mechanism 132 (FIG. 4).

In some embodiments and as described in further detail below, some components of the electronics assembly 134 are carried by the reusable portion 102 and some components are carried by the disposable portion 104 (both shown in FIGS. 1 and 4). For example, the disposable portion 104 may include an identifier 142 (for example, an RFID transmitter or EEPROM) to facilitate providing properties of the therapeutic agent 106 to the reusable portion 102. Such properties may include, for example, the type and/or volume of the therapeutic agent 106 carried by the syringe assembly 118. The reusable portion 102 may use the properties of the therapeutic agent 106 to determine, for example, if a patient associated with the reusable portion 102 is authorized to use, or has been prescribed, the therapeutic agent 106. As another example, a disposable portion may include securing device 144 that is operably coupled to the controller 136. The securing device 144 may initially inhibit the syringe assembly 118 from moving from the stowed configuration to the deployed configuration, and the controller 136 may actuate the securing device 144 to permit the syringe assembly 118 to move from the stowed configuration to the deployed configuration. In other embodiments, each of the components of the electronics assembly 134 is carried by the reusable portion 102. In some embodiments, the controller 136 is operatively coupled to one or more of the other components of the electronics assembly 134 by a wired connection. In some embodiments, the controller 136 is operatively coupled to one or more of the other components of the electronics assembly 134 by a wireless connection.

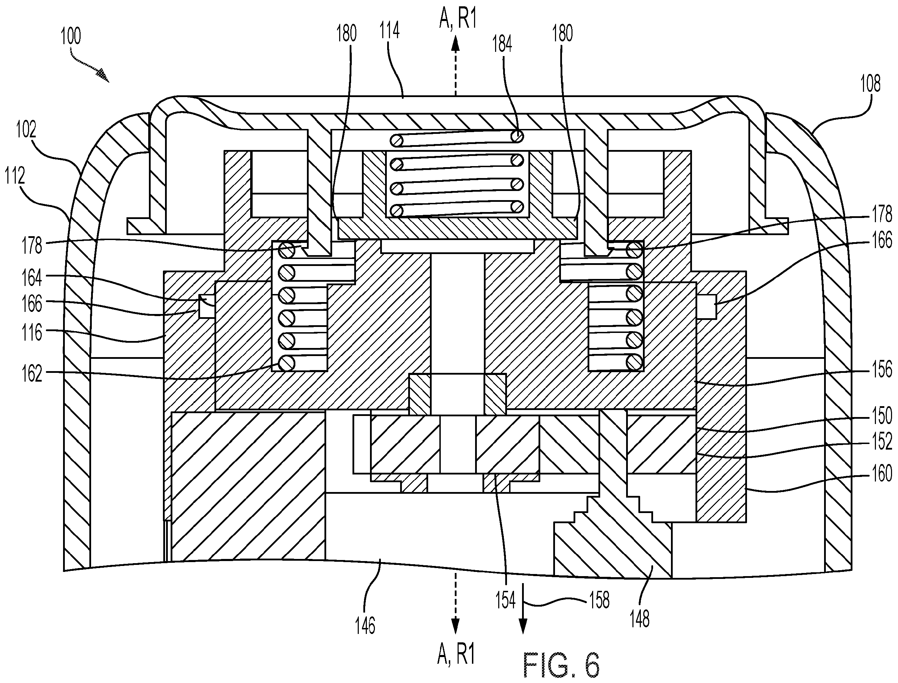

Referring now to FIGS. 6-8, the proximal end 108 of the device 100, specifically the user input 114 and a proximal portion of the drive mechanism 116, is shown in further detail. The drive mechanism 116 includes a carriage 146 (FIG. 6) that is translatably carried in the housing 112 of the reusable portion 102. The carriage 146 carries a first actuator 148 that operatively couples to the electronic controller 136 (FIG. 5). The first actuator 148 may be a rotary actuator, more specifically an electric motor, that drivably couples to a transmission or speed reducer. The actuator 148 drivably couples to a gear train 150, more specifically a first gear 152 that drivably couples to a second gear 154. The second gear 154 is fixed relative to a follower 156, and the follower 156 is rotatably carried by the carriage 146. As such, the carriage 146, the actuator 148, the gear train 150, and the follower 156 are translatable together within the housing 112 of the reusable portion 102. Illustratively, the carriage 146, the actuator 148, the gear train 150, and the follower 156 are translatable in a drive direction 158 (FIG. 6) that is substantially parallel to the longitudinal axis A of the device 100 (that is, parallel ± 10 degrees).

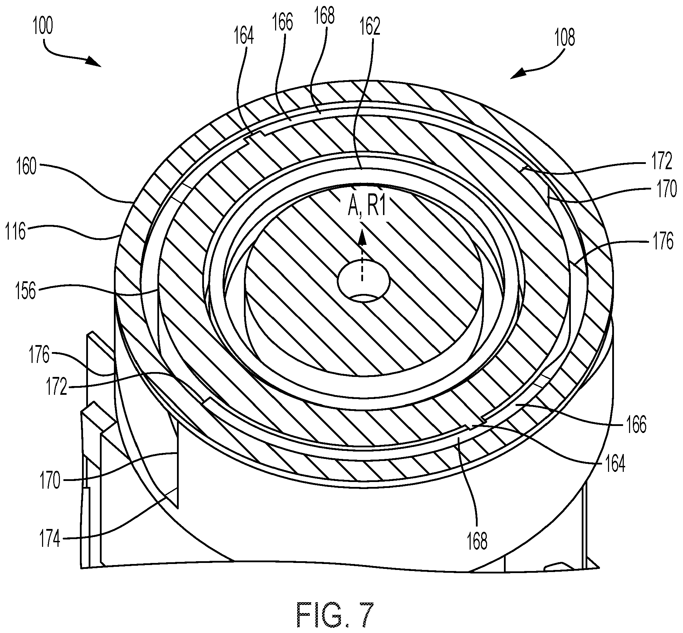

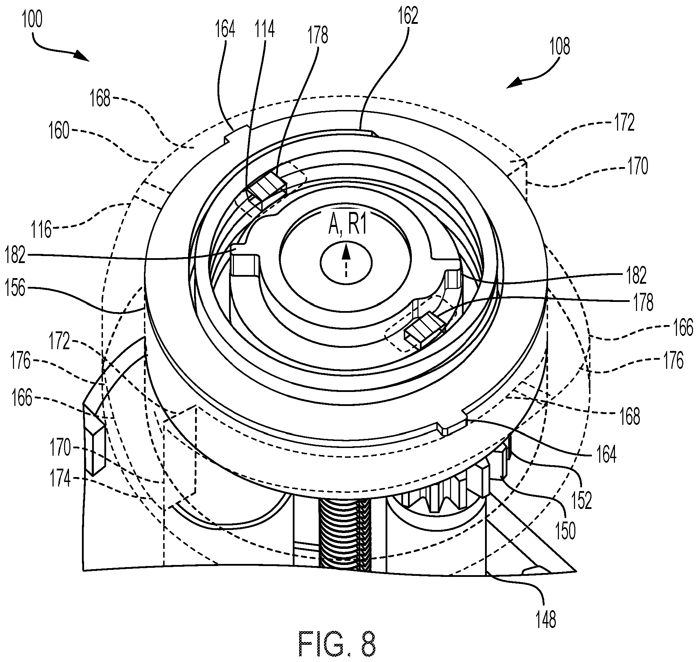

The follower 156 movably couples to a guide 160, and the guide 160 is fixed relative to the housing 112 of the reusable portion 102. A compression spring 162 urges the follower 156 distally and into engagement with the guide 160. Generally, the follower 156 and the guide 160 includes features that facilitate translating the follower 156 relative to the guide 160 as the follower 156 rotates relative to the guide 160 about a rotation axis R1 that is substantially parallel to the longitudinal axis A of the device 100 (that is, parallel ± 10 degrees). More specifically, the follower 156 includes two radially-outwardly extending protrusions 164 that move along an angular track 166, or generally proximally-facing wall, defined by the guide 160 as the follower 156 is rotated by the actuator 148. The protrusions 164 simultaneously move along two similar sections, or halves, of the track 166. Referring specifically to FIGS. 7 and 8, each half of the track 166 includes a plateau portion 168 that couples to a cliff portion 170 at an edge 172, a valley portion 174 coupled to the cliff portion 170 opposite the plateau portion 168, and a slope portion 176 coupled to the valley portion 174 opposite the cliff portion 170. Each slope portion 176 also couples to the plateau portion 168 of the other half of the track 166.

In the illustrated embodiment, the various portions of the track 166 are as follows. The cliff portions 170 are substantially parallel to the longitudinal axis A of the device 100 (that is, parallel ± 10 degrees). The plateau portions 168 and the valley portions 174 are substantially perpendicular to the longitudinal axis A of the device 100 (that is, perpendicular ± 10 degrees). The slope portions 176 extend helically relative to the longitudinal axis A of the device 100.

In other embodiments, the follower 156 and/or the guide 160 may have different forms. For example, the track 166 could have a different shape. More specifically, the track could include additional slope portions (not shown) instead of the cliff portions 170, and such slope portions could extend helically in the opposite directions as the slope portions 176. As another example, the follower 156 could include a different number of protrusions 164 and/or the guide 160 could include a track 166 with a different number of similar sections. As yet another example, the follower 156 could include a track 166 that movably receives one or more protrusions 164 formed on the guide 160.

With specific reference now to FIG. 6, the proximal end 108 of the device 100 also includes features for selectively inhibiting motion of the user input 114 relative to the housing 112 of the reusable portion 102 and, as a result, actuation of the user input 114. More specifically, the user input 114 includes one or more snap hooks 178 (two shown) that extend through openings 180 formed in the guide 160. The hooks 178 engage the guide 160 and hold the user input 114 in a depressed configuration relative to the housing 112. With specific reference now to FIGS. 6 and 8, the follower 156 includes legs 182 (FIG. 8) that engage and release the hooks 178 from the guide 160 as the follower 156 rotates relative to the guide 160. When the hooks 178 disengage the guide 160, a compression spring 184 expands and pushes the user input 114 to an elevated configuration relative to the housing 112. These features are described in further detail in the following paragraphs.

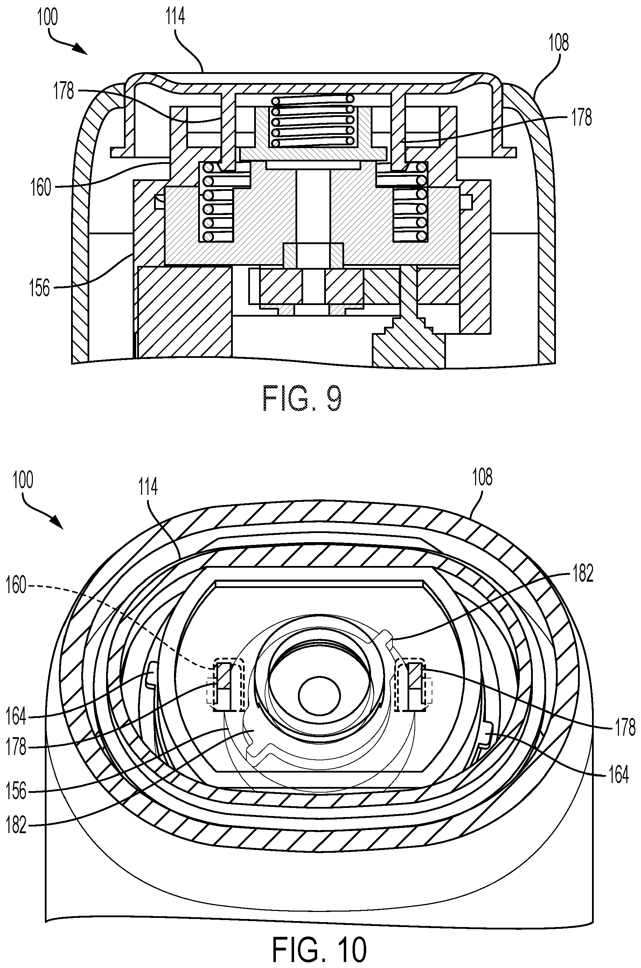

Referring to FIGS. 9-16, motion and several configurations of the various components at the proximal end 108 of the device 100 are as follows. Although the device 100 may remain in some of the configurations for certain periods of time, other configurations are shown for illustrative purposes, and the device 100 may simply transition through those configurations without remaining in them for a period of time. FIGS. 9 and 10 illustrate a first configuration of the components at the proximal end 108 of the device 100. In the first configuration, the snap hooks 178 of the user input 114 engage the guide 160 and hold the user input 114 in the depressed configuration, and the user input 114 is not actuatable by a user. In the first configuration, the legs 182 of the follower 156 (FIG. 10) are disposed apart from the hooks 178, and the protrusions 164 of the follower 156 (FIG. 10) are disposed in the plateau portions 168 of the guide 160 (FIG. 8).

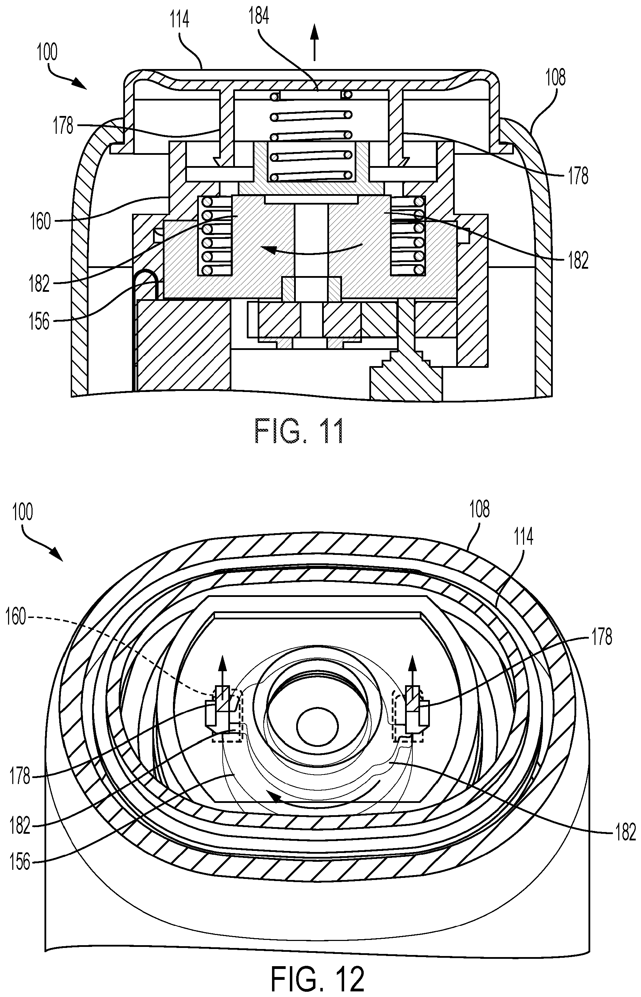

As shown in FIGS. 11 and 12, the first actuator 148 (FIG. 6) rotatably drives the follower 156 (illustratively, in a clockwise direction as viewed from the proximal end 108) such that the device 100 moves to a second configuration. Moving to the second configuration causes the legs 182 of the follower 156 to engage and release the hooks 178 of the user input 114 from the guide 160, and the compression spring 184 (FIG. 11) expands and pushes the user input 114 to the elevated configuration, wherein the user input is moved proximally from a distal position (depressed configuration in FIG. 9) to a position proximal to the distal position (elevated configuration in FIG. 11). However, the legs 182 of the follower 156 are aligned with the hooks 178, and the follower 156 thereby inhibits actuation of the user input 114. In the second configuration, the protrusions 164 of the follower 156 remain disposed in the plateau portions 168 of the guide 160 (all shown in FIG. 8).

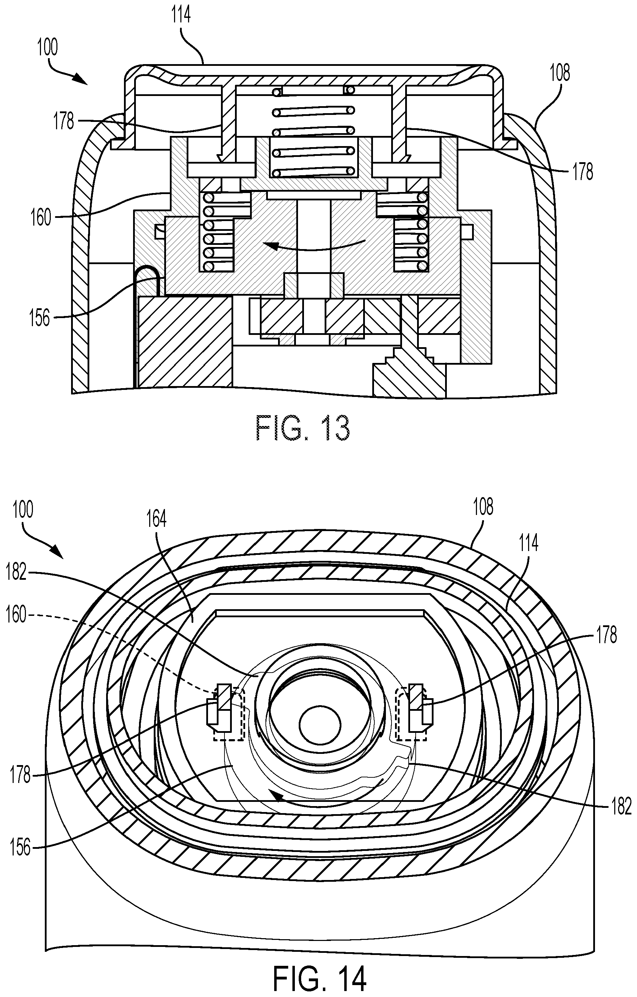

Next and as shown in FIGS. 13 and 14, the first actuator 148 (FIG. 6) rotatably drives the follower 156 (in the same direction - illustratively, the clockwise direction as viewed from the proximal end 108) such that the device 100 moves to a third configuration. In the third configuration, the legs 182 of the follower 156 are disposed apart from the hooks 178 of the user input 114, and the follower 156 thereby permits actuation of the user input 114. In the third configuration, the protrusions 164 of the follower 156 (one protrusion 164 being visible in FIG. 14) remain disposed in the plateau portions 168 of the guide 160 (FIG. 8).

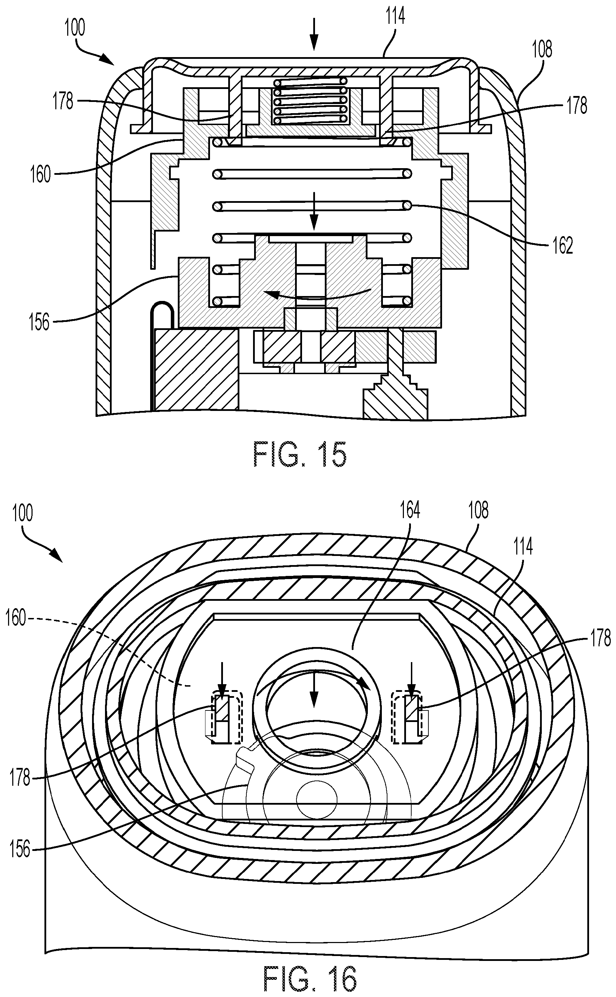

As shown in FIGS. 15 and 16, upon actuation of the user input 114, the first actuator 148 (FIG. 6) rotatably drives the follower 156 (in the same direction - illustratively, the clockwise direction as viewed from the proximal end 108) such that the device 100 moves to a fourth configuration. Moving to the fourth configuration causes the protrusions 164 of the follower 156 (one protrusion 164 being visible in FIG. 16) to move over the edges 172 and cliff portions 170 of the guide 160 (FIG. 8). This action permits the spring 162 (FIG. 15) to expand and distally push the follower 156 until the protrusions 164 of the follower 156 engage the valley portions 174 of the guide 160 (FIG. 8). The follower 156 thereby distally pushes the carriage 146 (FIG. 6). The carriage 146 pushes the syringe assembly 118 (FIGS. 3 and 4) from the stowed configuration to the deployed configuration, and the syringe assembly 118 may then deliver the therapeutic agent to the patient. Actuation of the user input 114 also reengages the snap hooks 178 of the user input 114 with the guide 160, which holds the user input 114 in the depressed configuration.

Although not specifically illustrated, the first actuator 148 then rotatably drives the follower 156 such that the device 100 moves to the first configuration. More specifically, the protrusions 164 of the follower 156 to move over the slope portions 176 of the guide 160 (FIG. 8) to move the follower 156. The syringe assembly 118 returns from the deployed configuration to the stowed configuration, and the disposable portion 104 may be replaced with a fresh disposable portion 104.

With continued general reference to FIGS. 9-16 and additional reference to FIG. 5, the electronics assembly 134 may cause the device 100 to transition through the above configurations upon detecting one or more conditions. For example, the sensors 140 of the electronics assembly 134 may include a proximity sensor for detecting detachment of one or more components of the disposable portion 104 from the device 100, such as a needle shield (shown elsewhere). Upon detecting detachment of the needle shield from the disposable portion 104, the device 100 may transition from the first configuration to the second configuration. As another example, the sensors 140 may include a contact sensor for detecting contact between the distal end 110 of the device 100 and the skin of the patient. Upon detecting contact with the skin of the patient, the device 100 may transition from the second configuration to the third configuration. In some embodiments, the electronics assembly 134 may facilitate illuminating the user input 114 to indicate that the user input 114 is ready for actuation. As yet another example, the sensors 140 may include a contact sensor or a proximity sensor for detecting actuation of the user input 114.

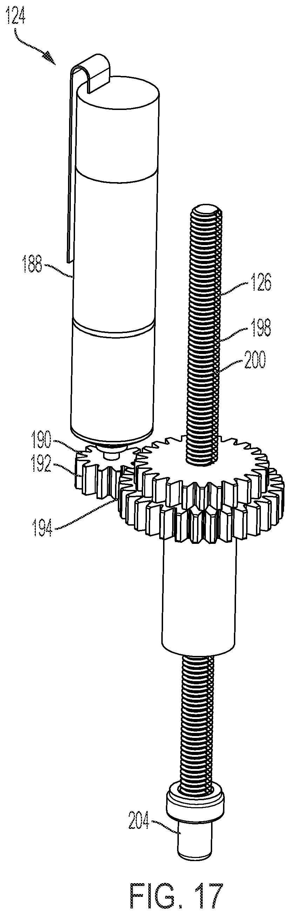

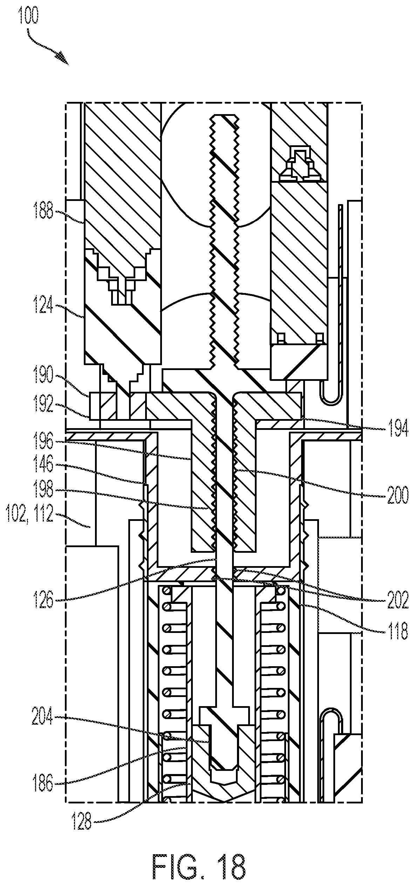

FIGS. 17 and 18 illustrate the therapeutic agent delivery mechanism 124 of the device 100. In the transverse sectional view of FIG. 18, the therapeutic agent delivery mechanism 124 is also illustrated adjacent other components of the device 100, such as the carriage 146, a syringe chamber 186, the piston 128.

With continued reference to FIGS. 17 and 18, the therapeutic agent delivery mechanism 124 is carried by and translates with the carriage 146 relative to the housing 112 of the reusable portion 102. The therapeutic agent delivery mechanism 124 includes a second actuator 188 that operatively couples to the electronic controller 136 (FIG. 5). The second actuator 188 may be a rotary actuator, more specifically an electric motor, that drivably couples to a transmission or speed reducer. The actuator 188 drivably couples to a gear train 190, more specifically a first gear 192 that drivably couples to a second gear 194. The second gear 194 includes internal threads 196 (FIG. 18) that couple to external threads 198 of the plunger 126. The plunger 126 is rotatably fixed but translatable relative to the carriage 146 (FIG. 18). Specifically, the plunger 126 couples to the carriage 146 via a key and slot interface, more specifically the plunger 126 includes external slots 200 that receive keys 202 (FIG. 18) formed on the carriage 146. The plunger 126 also includes a ram 204 for engaging the piston 128 of the syringe assembly 118.

With further reference to FIGS. 17 and 18, motion of the various components of the therapeutic agent delivery mechanism 124 is as follows. The actuator 188 is energized to rotatably drive the gear train 190 relative to the carriage 146. The plunger 126 thereby translates relative to the second gear 194 and the carriage 146. The plunger 126 distally pushes the piston 128 in the syringe chamber 186. As described above, such motion of the piston 128 causes the syringe assembly 118 to deliver the therapeutic agent from the needle (shown elsewhere).

With general reference to FIGS. 1-18, the controller 136 (FIG. 5) may actuate the drive mechanism 116 and the therapeutic agent delivery mechanism 124 sequentially upon detecting one or more conditions. More specifically, in some embodiments the drive mechanism 116 is actuated to move the syringe assembly 118 from the stowed configuration to the deployed configuration, and thereafter the therapeutic agent delivery mechanism 124 is actuated to drive the plunger 126 and the piston 128 and thereby deliver the therapeutic agent 106 from the needle 120. In these embodiments, the sensors 140 of the electronics assembly 134 may include a position sensor, such as an encoder (not shown) coupled to the actuator 148, for determining if the syringe assembly 118 is in the stowed configuration or the deployed configuration. Upon detecting that the syringe assembly 118 is in the deployed configuration, the therapeutic agent delivery mechanism 124 may be actuated to deliver the therapeutic agent 106 from the needle 120. After delivering the therapeutic agent 106, the drive mechanism 116 is actuated again to permit the syringe assembly 118 to move from the deployed configuration to the stowed configuration, and then the therapeutic agent delivery mechanism 124 is actuated again to retract the plunger 126 from the syringe assembly 118. More specifically, upon detecting that the syringe assembly 118 has returned to the stowed configuration, the therapeutic agent delivery mechanism 124 is actuated to retract the plunger 126 from the syringe assembly 118.

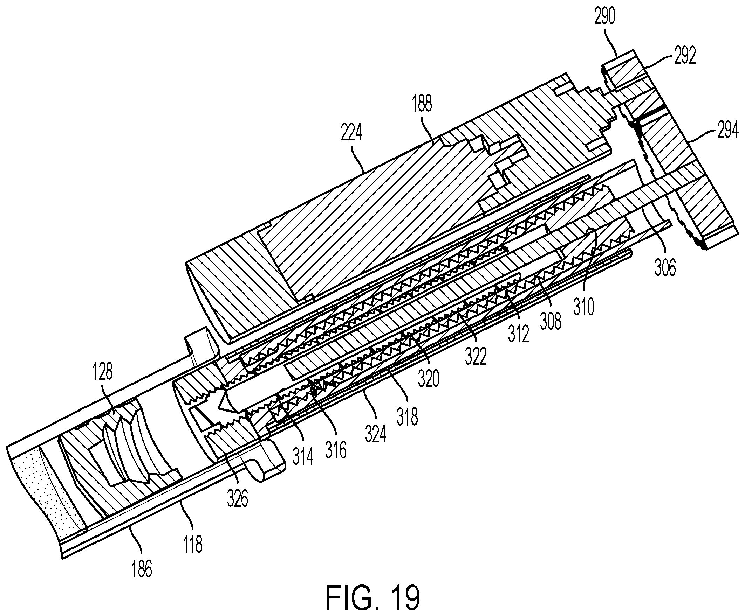

FIGS. 19-21 illustrate a therapeutic agent delivery mechanism 224 according to another exemplary embodiment of the present disclosure. The therapeutic agent delivery mechanism 224 may be used in place of the therapeutic agent delivery mechanism 124 of the therapeutic agent delivery device 100 (both shown elsewhere). As such, the therapeutic agent delivery mechanism 224 would be carried by the carriage 146 of the drive mechanism 116 (both shown elsewhere). The therapeutic agent delivery mechanism 224 and the drive mechanism 116 could also be operated using similar sequences as those described above. The therapeutic agent delivery mechanism 224 is also illustrated as being coupled to the syringe assembly 118, more specifically the syringe chamber 186 and the piston 128.

With continued reference to FIGS. 19-21, the therapeutic agent delivery mechanism 224 includes the second actuator 188 of the electronics assembly 134 (FIG. 5). The actuator 188 drivably couples to a gear train 290 (FIG. 19), more specifically a first gear 292 that drivably couples to a second gear 294. The second gear 294 drivably couples to a non-round drive shaft 306, more specifically a square drive shaft 306. The drive shaft 306 is rotatable relative to the carriage 146 of the drive mechanism 116 (both shown elsewhere) and translatably fixed relative to the carriage 146. The drive shaft 306 is rotatably driven by the gear train 290. The drive shaft 306 drivably couples to an intermediate shaft 308, and the drive shaft 306 is received in a non-round cross-sectional opening 310 of the intermediate shaft 308, more specifically a square cross-sectional opening 310. The intermediate shaft 308 is thereby rotatable together with the drive shaft 306 relative to the carriage, but translatable relative to the drive shaft 306. The intermediate shaft 308 includes external threads 312 and internal threads 314. The external threads 312 couple to internal threads 316 of an outer shaft 318, and the internal threads 316 couple to external threads 320 of an inner shaft or plunger 322. The outer shaft 318 is fixed relative to the carriage, for example, via a mounting bracket (not shown). The plunger 322 is rotatably fixed relative to the outer shaft 318 via a sleeve 324. More specifically, one or both of the plunger 322 and the outer shaft 318 couple to the sleeve 324 via key and slot interfaces (not shown). As a result, the plunger 322 and the outer shaft 318 are rotatably fixed relative to the sleeve 324 and, therefore, each other. The plunger 322 also includes a ram 326 for engaging the piston 128 of the syringe assembly 118.

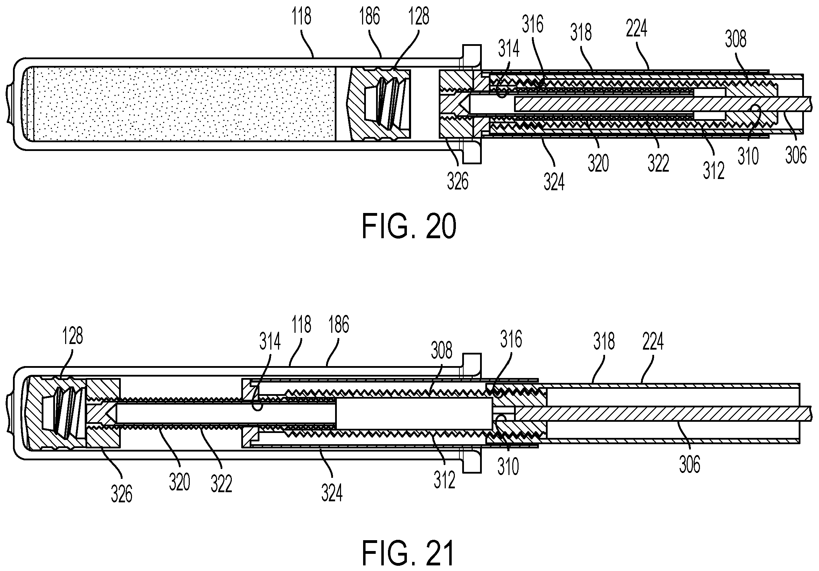

With further reference to FIGS. 19-21 and more specifically FIGS. 20 and 21, motion of the various components of the therapeutic agent delivery mechanism 224 is as follows. The rotary actuator 188 is energized to rotatably drive the gear train 290, the drive shaft 306, and the intermediate shaft 308 relative to the outer shaft 318 and the carriage (shown elsewhere). The intermediate shaft 308 thereby rotates and translates relative to the outer shaft 318, and the plunger 322 thereby translates relative to the intermediate shaft 308. The plunger 322 distally pushes the piston 128 in the syringe chamber 186. As described above, such motion of the piston 128 causes the syringe assembly 118 to deliver the therapeutic agent 106 from the needle 120 (both shown elsewhere).

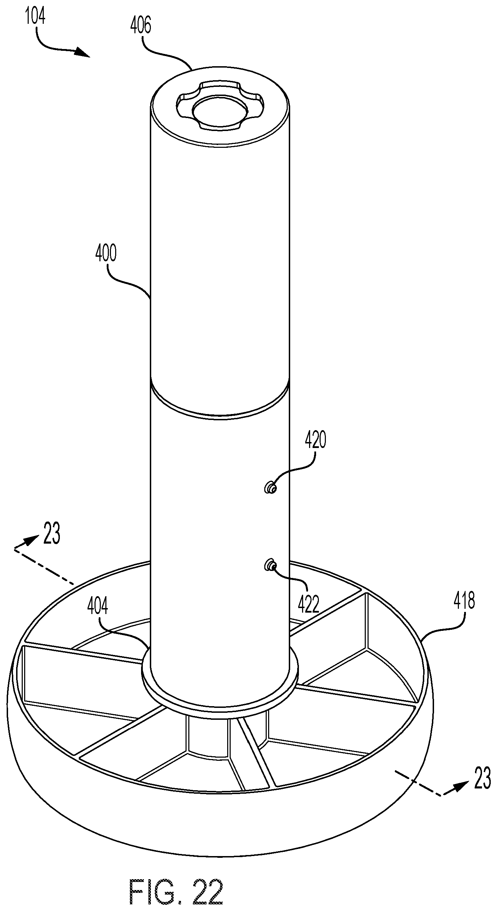

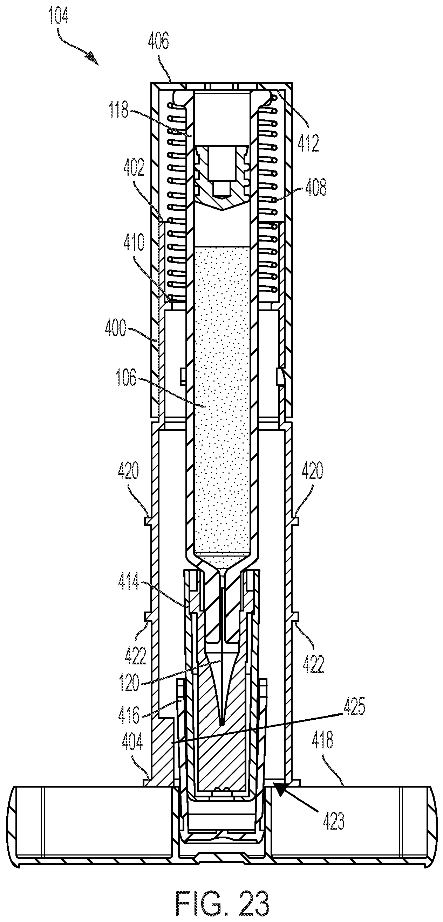

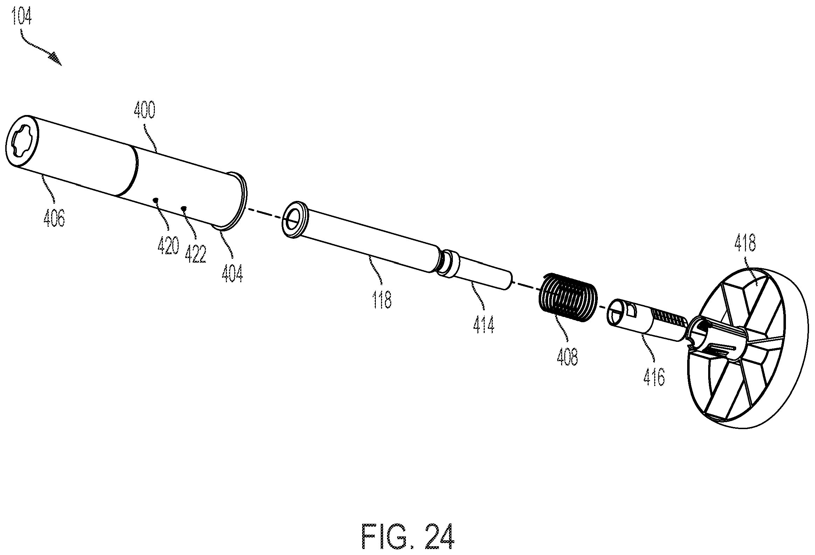

FIGS. 22-24 illustrate the disposable portion 104 of the device 100. With specific reference to the section view of FIG. 23 and the exploded view of FIG. 24, the disposable portion 104 includes a housing 400 having a proximal end 402 (FIG. 23) and a distal end 404. A proximal cap 406 covers the proximal end 402 of the housing 400. The proximal cap 406 is fixed relative to the syringe assembly 118 and movable relative to the housing 400, and the proximal cap 406 and the syringe assembly 118 are thereby movable together relative to the housing 400. As a result, the drive mechanism 116 (FIG. 6) distally pushes the proximal cap 406 to cause the syringe assembly 118 to move from the stowed configuration to the deployed configuration.

With continued reference to FIGS. 23 and 24, internally the disposable portion 104 includes a compression spring 408. The compression spring 408 is compressed between an internal flange 410 (FIG. 23) in the housing 400 and an internal end wall 412 (FIG. 23) of the proximal cap 406. The compression spring 408 biases the syringe assembly 118 and the proximal cap 406 away from the deployed configuration and toward the stowed configuration. As a result, the compression spring 408 pushes the syringe assembly 118 and the proximal cap 406 from the deployed configuration to the stowed configuration after the needle 120 delivers the therapeutic agent 106 (FIG. 23) and the drive mechanism 116 (FIG. 6) moves proximally in the housing 112 of the reusable portion 102 (FIG. 3).

At the distal end 404 of the housing 400, the disposable portion 104 carries a needle shield 414, a shield puller 416, and a base cap 418. The needle shield 414 the shield puller 416, and the base cap 418 initially obscures the needle 120 of the syringe assembly 118. The needle shield 414, the shield puller 416, and the base cap 418 are fixed relative to each other and together detachable from the housing 400 to facilitate delivering the therapeutic agent 106 from the needle 120. The distal end 404 defines an axial opening 423 in which a proximal portion of the base cap 418 is inserted therein. A rib 425 can be disposed along an interior surface of the housing 400 and extends radially inward. The rib may extend axially from the distal surface The radial extent of the rib 425 is sized to reduce the cross-sectional area of the axial opening 423 relative to the size of the axial opening without such rib. More than one rib 425 may be included and arranged, such as, for example, having the ribs circumferentially spaced apart from one another. The smaller cross-sectional area defined by the one or more ribs 425 may inhibit an object of certain sizes from entering into the axial opening 423 after the base cap 418 is removed.

With specific reference to FIG. 24, the housing 400 also includes features for securing the disposable portion 104 to the reusable portion 102 via the securing mechanism 132 (both shown in FIGS. 3). More specifically, the housing 400 includes one or more protrusions 420 and one or more magnetic elements 422 (for example, one or more permanent magnets) for coupling to the securing mechanism 132 and the reusable portion 102. These features are described in further detail below.

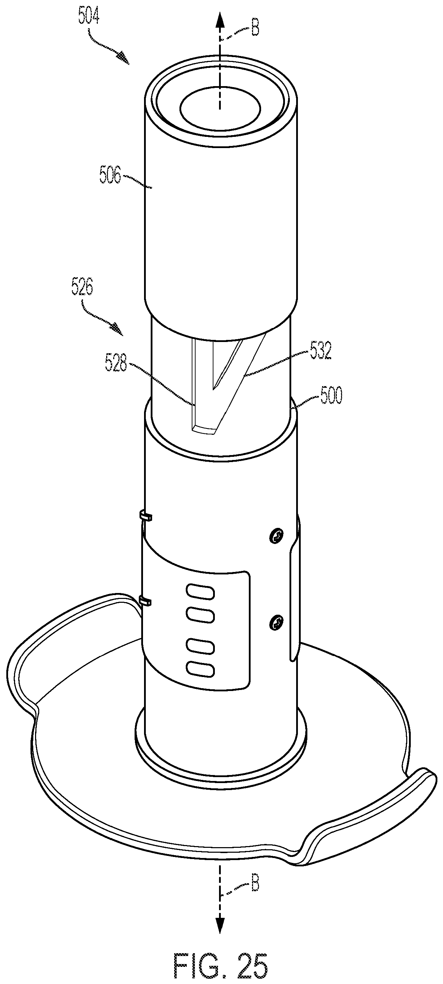

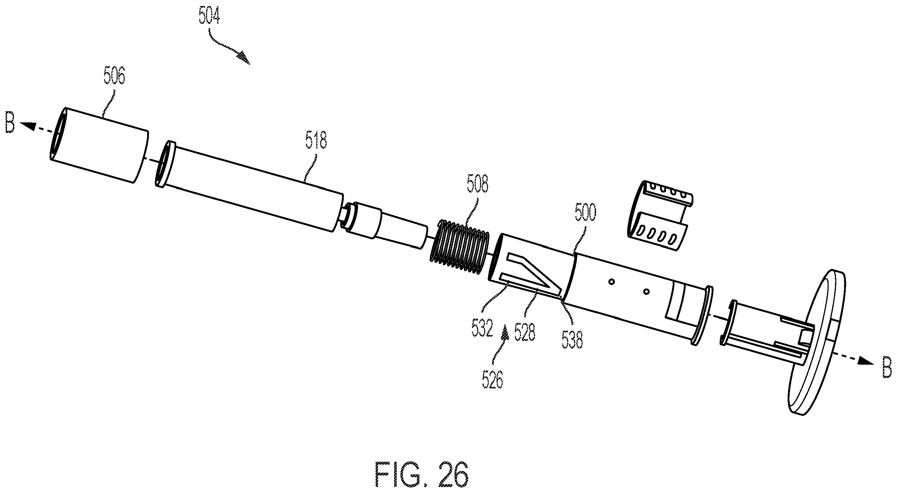

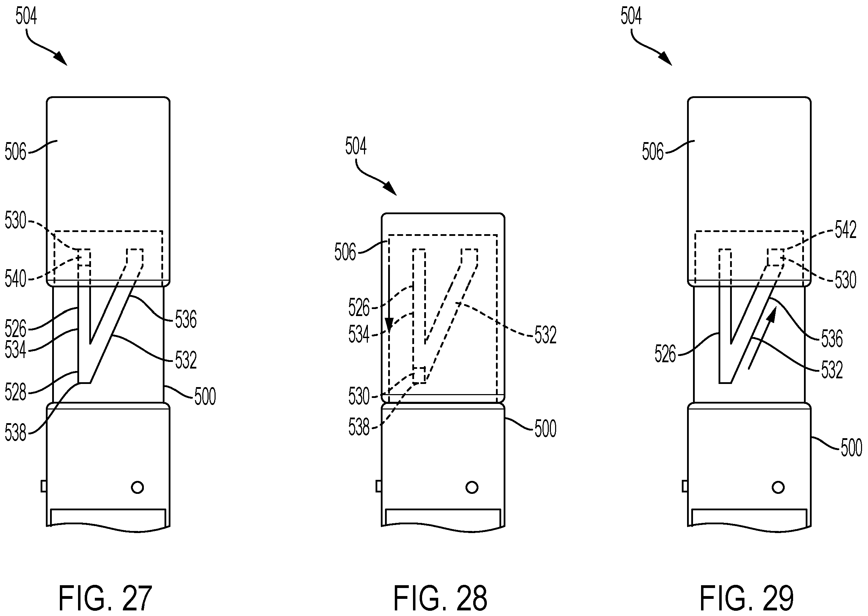

FIGS. 25-27 illustrate a disposable portion 504 according to another exemplary embodiment of the present disclosure. The disposable portion 504 may be used in place of the disposable portion 104 of the device 100 (both shown elsewhere). The disposable portion 504 is generally similar to the disposable portion 104, and similar features are identified with similar reference numbers. In contrast to the disposable portion 104, the disposable portion 504 further includes a mechanism 526 for locking out the disposable portion 504 after delivering the therapeutic agent (not shown). More specifically, the lock-out mechanism 526 permits the syringe assembly 518 (FIG. 26) to move from the stowed configuration to the deployed configuration and return to the stowed configuration only once. In the illustrated embodiment, the lock-out mechanism 526 includes a guide 528 defined by the housing 500 and one or more protrusions 530 (illustratively, three protrusions 530, one of which is shown in FIG. 27) defined by the proximal cap 506. The guide 528 includes one or more tracks 532 (illustratively, three tracks 532), or slots, and each of the tracks 532 movably receives one of the protrusions 530. Each track 532 includes a first track portion 534 and a second track portion 536. The first track portion 534 extends substantially parallel to a longitudinal axis B of the disposable portion 104 (that is, that is, parallel ± 10 degree). The first track portion 534 couples to the second track portion 536 at a distal end 538, and the second track portion 536 extends helically relative to the longitudinal axis B.

With reference to FIGS. 27-29, operation of the lock-out mechanism 526 is as follows. As shown in FIG. 27, each protrusion 530 is received at a proximal end 540 of one of the first track portions 534 when the syringe assembly 518 (FIG. 26) is initially in the stowed configuration. As shown in FIG. 28, when the proximal cap 506 and the syringe assembly 518 (FIG. 26) translate distally relative to the housing 500 and the syringe assembly 518 moves to the deployed configuration, each protrusion 530 moves along the first track portion 534 and arrives at the distal end 538 of the associated track 532. As shown in FIG. 29, when the cap 506 and the syringe assembly 518 (FIG. 26) translate proximally relative to the housing 500 and the syringe assembly 518 returns to the stowed configuration, the cap 506 pivots relative to the housing 500, for example, due to torsional energy stored by the compression spring 508 (FIG. 26). As a result, each protrusion 530 moves along the second track portion 536 and arrives at a proximal end 542 of the second track portion 536 of the associated track 532. When each protrusion 530 arrives at the proximal end 542 of the second track portion 536, each protrusion 530 may be inhibited from moving away from the proximal end 542, for example, by snap features (not shown) and/or interference between each protrusion 530 and the associated track 532.

In other embodiments, the lock-out mechanism 526 may take other forms. For example, the proximal cap 506 could define a guide including one or more tracks, and the housing 500 could include one or more protrusions that are movably received by the tracks 532.

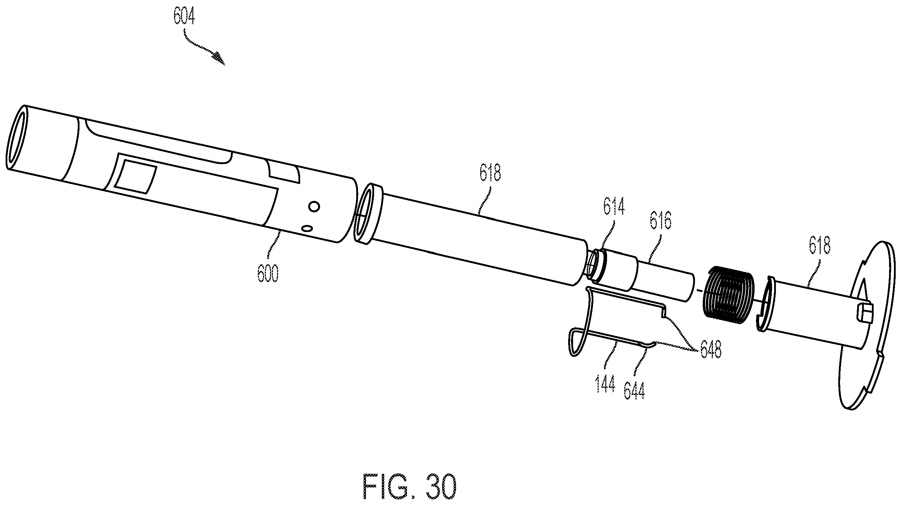

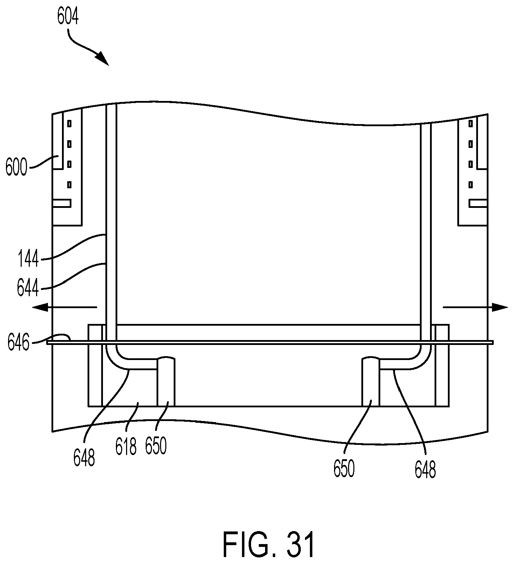

FIGS. 30 and 31 illustrate a disposable portion 604 according to yet another exemplary embodiment of the present disclosure. The disposable portion 604 may be used in place of the disposable portion 104 of the device 100 (both shown elsewhere). The disposable portion 604 is generally similar to the disposable portion 104, and similar features are identified with similar reference numbers. In contrast to the disposable portion 104, the disposable portion 604 further includes the securing device 144 of the electronics assembly 134 (FIG. 5), as briefly described above. Generally, the securing device 144 initially inhibits the base cap 618, and as a result the shield puller 614 and the needle shield 616 (both shown in FIG. 30), from being detached from the housing 600. The securing device 144 thereby inhibits the syringe assembly 618 from moving from the stowed configuration to the deployed configuration. The controller 136 (FIG. 5) actuates the securing device 144 to permit the syringe assembly 618 to move from the stowed configuration to the deployed configuration. In some embodiments, the controller 136 actuates the securing device 144 upon determining that one or more conditions have been met. For example, the controller 136 may actuate the securing device 144 upon determining that the disposable portion 604 has been coupled to the reusable portion 102 (FIGS. 1-18) and/or determining that a patient associated with the reusable portion 102 has been prescribed the therapeutic agent (not shown).

With continued reference to FIGS. 30 and 31, the securing device 144 illustratively includes a retainer 644, more specifically a shaped wire, and a release device 646 (FIG. 31), more specifically a wire constructed of one or more shape memory materials. The retainer 644 is carried by and partially extends around the housing 600. The retainer 644 includes one or more transversely extending feet 648 (illustratively, two feet 648). The base cap 618 includes one or more apertures 650 (illustratively, two apertures 650 - FIG. 31), and each of the apertures 650 initially receives one of the feet 648. The release device 646 is also carried by and extends at least partially around the housing 600. The release device 646 also extends proximate the feet 648 of the retainer 644. The release device 646 is actuatable, more specifically contractable, for example, by receiving thermal energy from the controller 136 (FIG. 5). Actuation causes the release device 646 to pull the feet 648 of the retainer 644 from the apertures 650 of the base cap 618. This action permits the base cap 618, the shield puller 614, and the needle shield 616 to be detached from the disposable portion 604, which permits the therapeutic agent to be delivered from the needle (not shown).

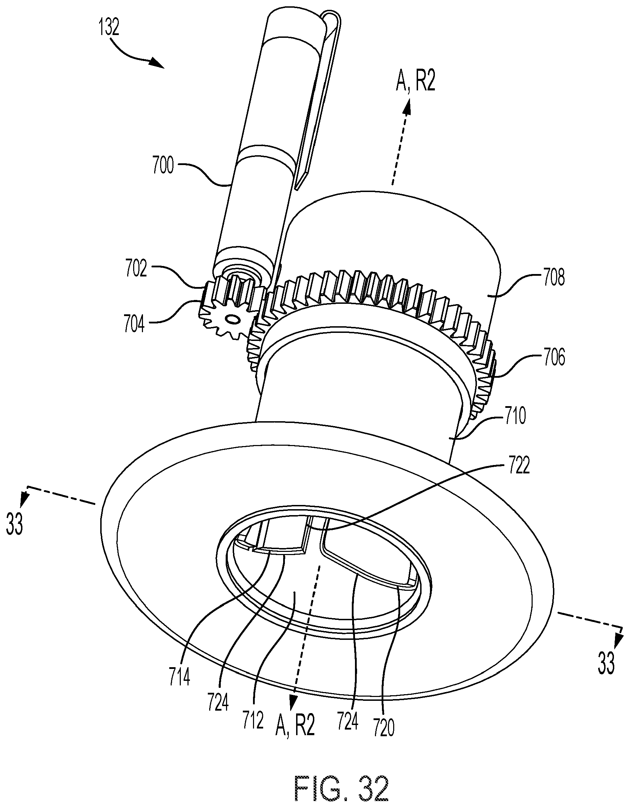

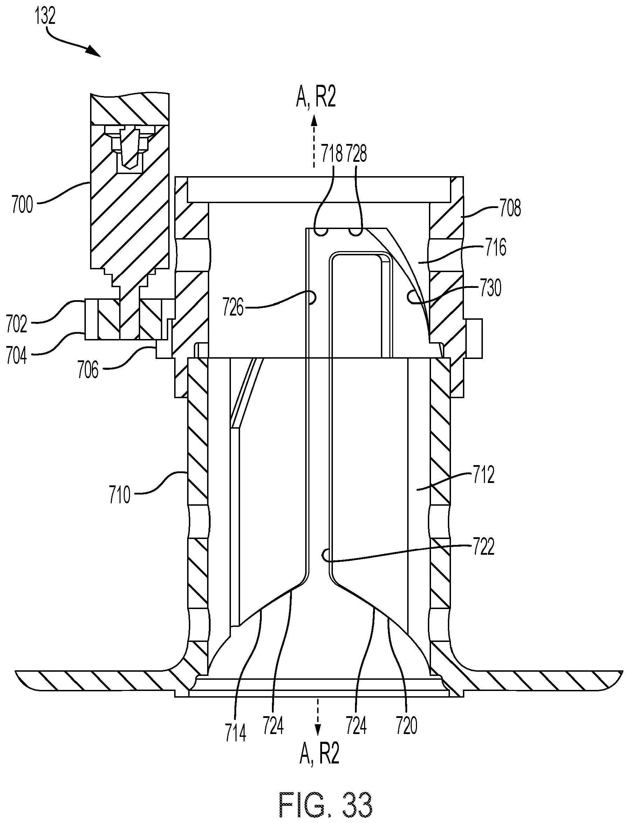

FIGS. 32 and 33 illustrate the securing mechanism 132 of the device 100. The securing mechanism 132 is carried within the housing 112 of the reusable portion 102 and adjacent to the distal end 110 (all shown in FIG. 3). The securing mechanism 132 includes a third actuator 700 that operatively couples to the electronic controller 136 (FIG. 5). The third actuator 700 may be a rotary actuator, more specifically an electric motor, that drivably couples to a transmission or speed reducer. The actuator 700 drivably couples to a gear train 702, more specifically a first gear 704 that drivably couples to a second gear 706. The second gear 706 is monolithically formed with, or otherwise fixed relative to, a proximal cylinder 708. The proximal cylinder 708 is rotatably coupled to a distal cylinder 710, which is fixed relative to relative to the housing 112 of the reusable portion 102. The proximal cylinder 708 rotates relative to the distal cylinder 710 about a securing rotation axis R2 that is substantially parallel to the longitudinal axis A of the device 100 (that is, parallel ± 10 degrees).

With continued reference to FIGS. 32 and 33, the proximal cylinder 708 and the distal cylinder 710 include features that facilitate selectively securing the disposable portion 104 to the reusable portion 102 (both shown in FIG. 3). More specifically, an internal surface 712 of the distal cylinder 710 includes one or more leading tracks 714 (illustratively, two leading tracks 714 - only one leading track 714 is visible in FIGS. 32 and 33), or slots, for receiving the protrusions 420 of the disposable portion 104. Similarly, an internal surface 716 of the proximal cylinder 708 (FIG. 33) includes one or more securing tracks 718 (illustratively, two securing tracks 718 - only one securing track 718 is visible in FIG. 33), or slots, for receiving the protrusions 420 of the disposable portion 104. As described in further detail below, the proximal cylinder 708 rotates relative to the distal cylinder 710 to selectively align and misalign the securing tracks 718 with the leading tracks 714. These actions permit and inhibit, respectively, the protrusions 420 of the disposable portion 104 to move between the leading tracks 714 and the securing tracks 718, which facilitates selectively securing the disposable portion 104 to the reusable portion 102.

With further reference to FIGS. 32 and 33, each leading track 714 of the distal cylinder 710 includes an inverted general funnel shape. More specifically, each leading track 714 includes a tapering distal portion 720 and a relatively narrow proximal portion 722. Each distal portion 720 tapers in width proceeding proximally. More specifically, each distal portion 720 includes two oppositely helically extending walls 724 that couple to the proximal portion 722. As a result, the walls 724 are configured to direct the protrusions 420 of the disposable portion 104 toward the proximal portion 722 as the disposable portion 104 is coupled to the reusable portion 102. The proximal portion 722 of each leading track 714 may have a width that is slightly larger than the width of the protrusions 420 of the disposable portion 104. The proximal portion 722 of each leading track 714 may be substantially parallel to the longitudinal axis A (that is, parallel ± 10 degrees).

With specific reference to FIG. 33, each securing track 718 of the proximal cylinder 708 includes an entry portion 726, a securing portion 728, and an egress portion 730. The entry portion 726 of each securing track 718 is selectively alignable with one of the leading tracks 714 to facilitate receiving the protrusions 420 of the disposable portion 104 therefrom. The entry portion 726 of each securing track 718 may be substantially parallel to the longitudinal axis A (that is, parallel ± 10 degrees). As such, the entry portion 726 may be referred to as a longitudinal portion. Opposite the leading track 714, the entry portion 726 of each securing track 718 couples to the securing portion 728. The securing portion 728 may be substantially perpendicular to the longitudinal axis A (that is, perpendicular ± 10 degrees). As such, the securing portion 728 may be referred to as a transverse portion. Opposite the entry portion 726, the securing portion 728 of each securing track 718 couples to the egress portion 730. The egress portion 730 of each securing track 718 may extend helically relative to the longitudinal axis A and away from the securing portion 728. As such, the egress portion 730 may be referred to as a helical portion. The egress portion 730 is selectively alignable with one of the leading tracks 714 to facilitate transferring the protrusions 420 of the disposable portion 104 thereto.

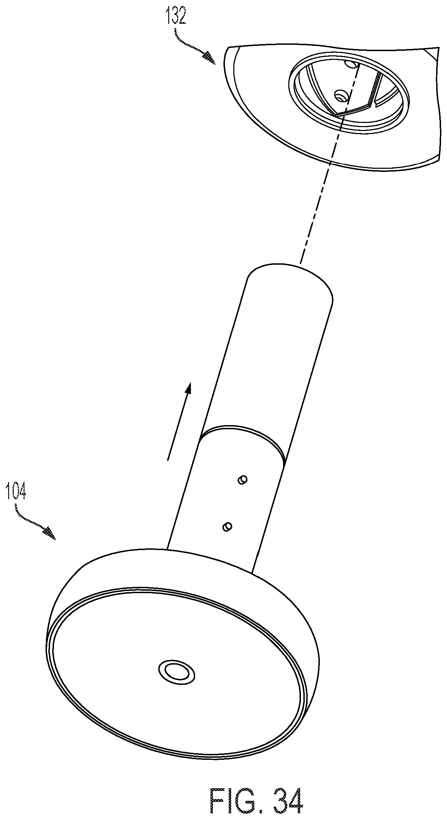

Referring to FIGS. 34-39, motion and several configurations of the securing mechanism 132 and the disposable portion 104 are as follows. Although the securing mechanism 132 and the disposable portion 104 may remain in some of the configurations for certain periods of time, other configurations are shown for illustrative purposes, and the securing mechanism 132 and the disposable portion 104 may simply transition through those configurations without remaining in them for a period of time. FIG. 34 illustrates a first or initial configuration of the securing mechanism 132 and the disposable portion 104. In the first configuration, the disposable portion 104 is detached from the securing mechanism 132 and the reusable housing 112 (FIG. 3).

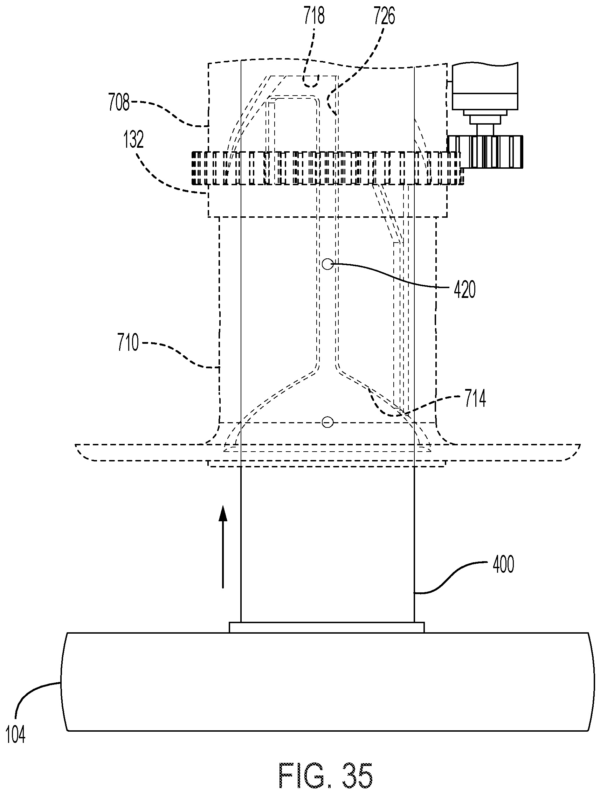

The disposable portion 104 is advanced proximally and toward the securing mechanism 132 to arrive in a second configuration, as shown in FIG. 35. In the second configuration, the housing 400 of the disposable portion 104 is received in the distal cylinder 710 and the proximal cylinder 708. In the second configuration, the protrusions 420 of the disposable portion 104 (one protrusion 420 being visible in FIG. 35) have also entered the leading tracks 714 of the distal cylinder 710 (one leading track 714 being visible in FIG. 35). In the second configuration, the leading tracks 714 of the distal cylinder 710 are aligned with the entry portions 726 of the securing tracks 718 of the proximal cylinder 708 (one entry portion 726 being visible in FIG. 35).

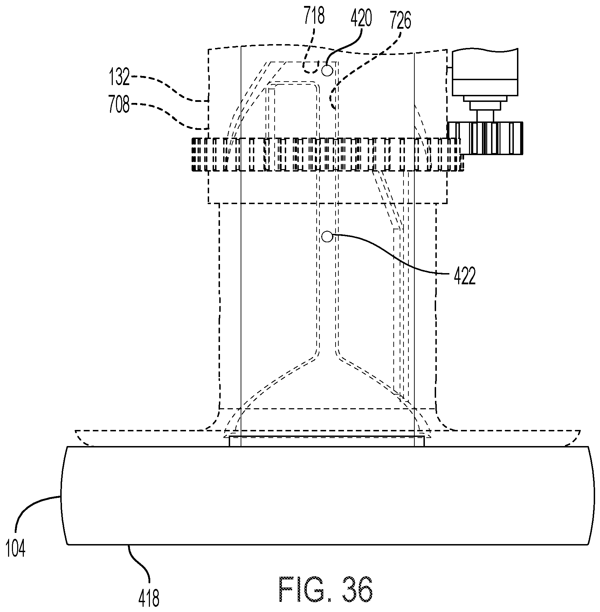

The disposable portion 104 is advanced further proximally to arrive in a third configuration, as shown in FIG. 36. In the third configuration, the base cap 418 of the disposable portion 104 abuts the securing mechanism 132 and the distal end 110 of the device 100 (shown elsewhere). In the third configuration, the protrusions 420 of the disposable portion 104 (one protrusion 420 being visible in FIG. 36) have also traversed the entry portions 726 of the securing tracks 718 of the proximal cylinder 708 (one entry portion 726 and one securing track 718 being visible in FIG. 36). The magnetic elements 422 of the disposable portion 104 (one magnetic element 422 being visible in FIG. 36) may also magnetically couple with one or more magnetic elements of the reusable portion 102 (for example, a ferromagnetic portion of the housing 112 or a ferromagnetic component carried in the housing) to hold the disposable portion 104 in the third configuration.

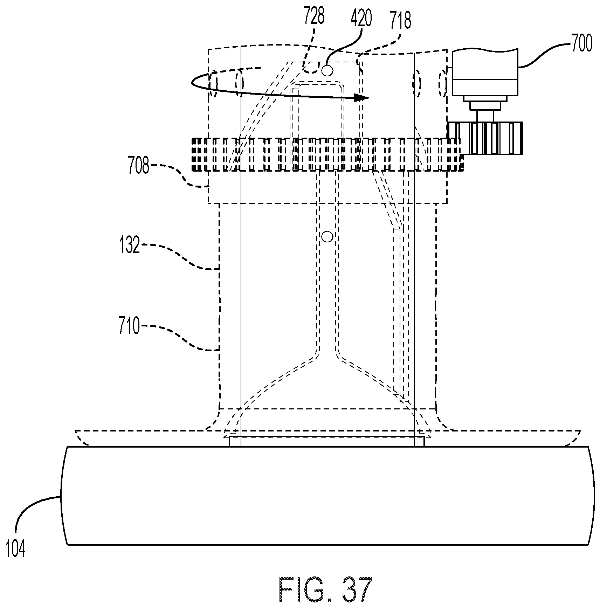

As shown in FIG. 37, the third actuator 700 is energized, and the proximal cylinder 708 rotates relative to the distal cylinder 710 (illustratively, in a counterclockwise direction as viewed from the proximal end 108 of the device 100 - shown elsewhere) such that the securing mechanism 132 moves to a fourth configuration. In the fourth configuration, the protrusions 420 of the disposable portion 104 (one protrusion 420 being visible in FIG. 37) are disposed in the securing portions 728 of the securing tracks 718 of the proximal cylinder 708 (one securing portion 728 and one securing track 718 being visible in FIG. 37). This inhibits detachment of the disposable portion 104 from the reusable portion 102, and the securing mechanism 132 may remain in the fourth configuration until the therapeutic agent 106 is delivered from the syringe assembly 118 (both shown in FIG. 3).

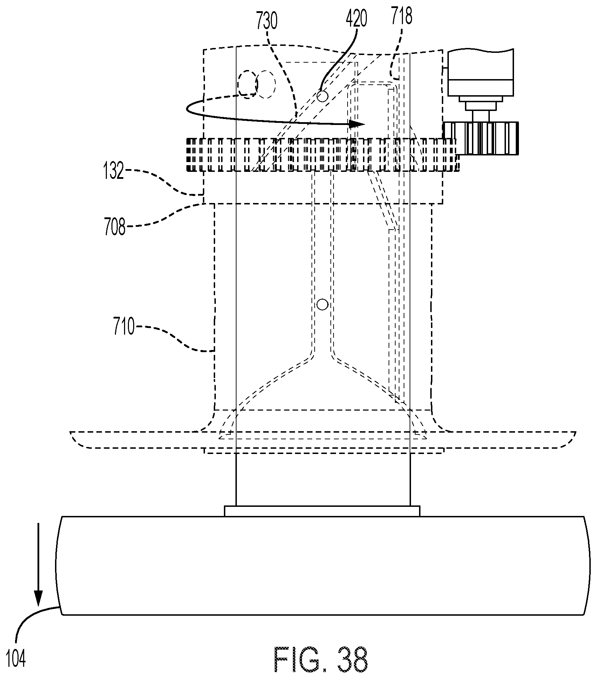

As shown in FIG. 38, the proximal cylinder 708 then rotates relative to the distal cylinder 710 (in the same direction - illustratively, in the counterclockwise direction as viewed from the proximal end 108) such that the securing mechanism 132 moves to a fifth configuration. In the fifth configuration, the protrusions 420 of the disposable portion 104 (one protrusion 420 being visible in FIG. 38) are disposed in the egress portions 730 of the securing tracks 718 of the proximal cylinder 708 (one egress portion 730 and one securing track 718 being visible in FIG. 38). As a result, the proximal cylinder 708 has begun pushing the protrusions 420 and the disposable portion 104 distally to eject the disposable portion 104.

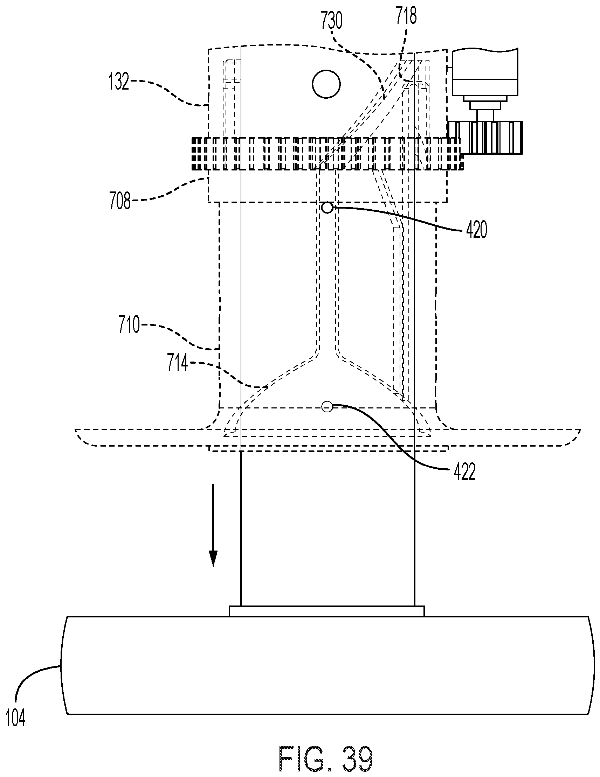

The proximal cylinder 708 further rotates relative to the distal cylinder 710 (in the same direction - illustratively, in the counterclockwise direction as viewed from the proximal end 108) to arrive in a sixth configuration, as shown in FIG. 39. In the sixth configuration, the egress portions 730 of the securing tracks 718 of the proximal cylinder 708 (one egress portion 730 and one securing track 718 being visible in FIG. 39) are aligned with the leading tracks 714 of the distal cylinder 710 (one leading track 714 being visible in FIG. 39). In the sixth configuration, the protrusions 420 of the disposable portion 104 (one protrusion 420 being visible in FIG. 38) are disposed in the leading tracks 714 of the distal cylinder 710. The magnetic elements 422 may also magnetically couple with one or more magnetic elements of the reusable portion 102 hold the disposable portion 104 in the sixth configuration, although a user may pull the disposable portion 104 distally to detach the disposable portion 104 from the securing mechanism 132 and the reusable portion 102.

With continued general reference to FIGS. 34-39 and additional reference to FIG. 5, the electronics assembly 134 may cause the securing mechanism 132 and the disposable portion 104 to transition through the above configurations upon detecting one or more conditions. For example, the sensors 140 of the electronics assembly 134 may include a proximity sensor for detecting the position of the disposable portion 104 relative to the reusable portion 102. Such a sensor may be, for example, a magnetic field sensor, such as a Hall effect sensor, for detecting the magnetic elements 422 of the disposable portion 104. Upon detecting that the disposable portion 104 has been inserted into the reusable portion 102 and disposed in the third configuration (FIG. 36), the securing mechanism 132 may transition to the fourth configuration (FIG. 37) to secure the disposable portion 104 to the reusable portion 102. As another example, the sensors 140 of the electronics assembly 134 may include a proximity sensor for detecting the position of the syringe assembly 118, more specifically whether the syringe assembly 118 is in the deployed configuration or the stowed configuration. Upon detecting that the syringe assembly 118 has moved from the deployed configuration to the stowed configuration (that is, the syringe assembly 118 has delivered the therapeutic agent 106 to the patient), the securing mechanism 132 may transition from the fourth configuration (FIG. 37) to the fifth configuration (FIG. 38) and the sixth configuration (FIG. 39) to facilitate detaching the disposable portion 104 from the reusable portion 102. Alternatively, the controller 136 may transition the securing mechanism 132 from the fourth configuration to the fifth configuration and the sixth configuration after a predetermined time period following actuation of the user input 114 (FIG. 3). The predetermined time period may be, for example, based on the typical time period for delivering the therapeutic agent 106 to a patient.

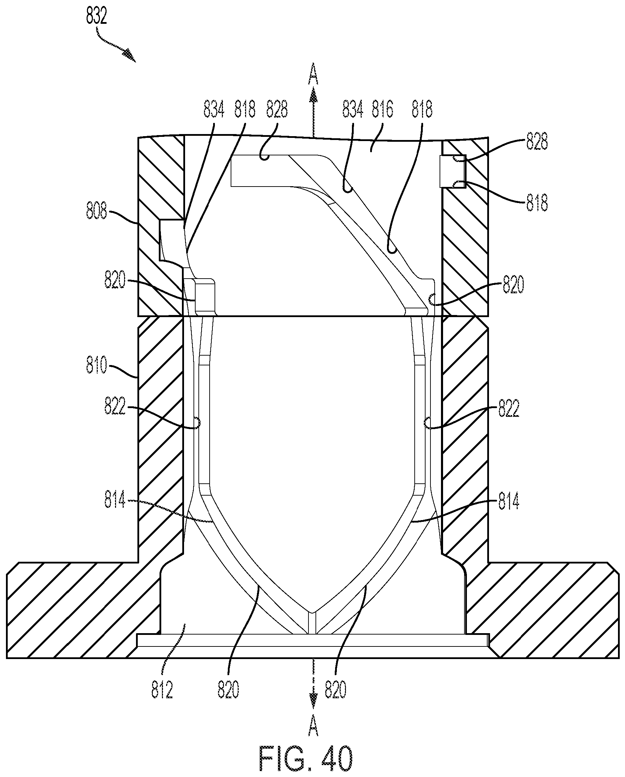

The securing mechanism 132 may be modified in various manners. For example, the securing mechanism 132 could include one or more protrusions, and the disposable portion 104 could include one or more tracks for receiving the protrusions. As another example and referring now to FIGS. 40-46, a securing mechanism 832 according to another exemplary embodiment of the present disclosure is illustrated. The securing mechanism 832 may be used in place of the securing mechanism 132. As such, the securing mechanism 832 would be carried by the housing 112 of the reusable portion 102 (both shown elsewhere). The securing mechanism 832 is generally similar to the securing mechanism 132, and similar features are identified with similar reference numbers.

With specific reference to FIG. 40, the securing mechanism 832 includes a distal cylinder 810 and a proximal cylinder 808 having different leading tracks 814 and securing tracks 818 than those of the distal cylinder 810 and the proximal cylinder 808, respectively. Specifically, the internal surface 812 of the distal cylinder 810 includes three leading tracks 814 (only two leading tracks 814 are visible in FIG. 40), and the internal surface 816 of the proximal cylinder 808 includes three securing tracks 818. Each leading track 814 of the distal cylinder 810 includes an inverted general funnel shape. More specifically, each leading track 814 includes a tapering distal portion 820 and a relatively narrow proximal portion 822. Each distal portion 820 tapers in width proceeding proximally. Each securing track 818 of the proximal cylinder 808 includes a distal portion 820, an entry/egress portion 834, and a securing portion 828. The distal portion 820 of each securing track 818 is selectively alignable with one of the leading tracks 814 to facilitate receiving the protrusions 420 of the disposable portion 104 therefrom. The distal portion 820 of each securing track 818 couples to the entry/egress portion 834. The entry/egress portion 834 of each securing track 818 may extend helically relative to the longitudinal axis A. As such, the entry/egress portion 834 may be referred to as a helical portion. Opposite the distal portion 820, the entry/egress portion 834 of each securing track 818 couples to the securing portion 828. The securing portion 828 may be substantially perpendicular to the longitudinal axis A (that is, perpendicular ± 10 degrees). As such, the securing portion 828 may be referred to as a transverse portion. The securing portion 828 terminates opposite the entry/egress portion 834.

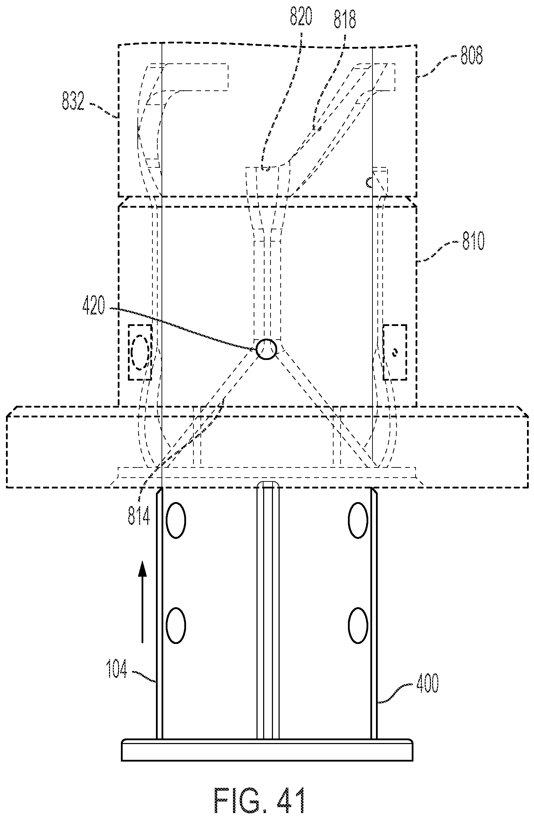

Referring to FIGS. 41-46, motion and several configurations of the securing mechanism 832 and the disposable portion 104 are as follows. Although the securing mechanism 832 and the disposable portion 104 may remain in some of the configurations for certain periods of time, other configurations are shown for illustrative purposes, and the securing mechanism 832 and the disposable portion 104 may simply transition through those configurations without remaining in them for a period of time. Although not specifically illustrated, in a first or initial configuration the disposable portion 104 is detached from the securing mechanism 832 and the reusable housing 112 (FIG. 3).

The disposable portion 104 is advanced proximally and toward the securing mechanism 832 to arrive in a second configuration, as shown in FIG. 41. In the second configuration, the housing 400 of the disposable portion 104 is received in the distal cylinder 810 and the proximal cylinder 808. In the second configuration, the protrusions 420 of the disposable portion 104 (one protrusion 420 being visible in FIG. 41) have also entered the leading tracks 814 of the distal cylinder 810 (one entire leading track 814 being visible in FIG. 41). In the second configuration, the leading tracks 814 of the distal cylinder 810 are aligned with the distal portions 820 of the securing tracks 818 of the proximal cylinder 808 (one entire securing track 818 being visible in FIG. 41).

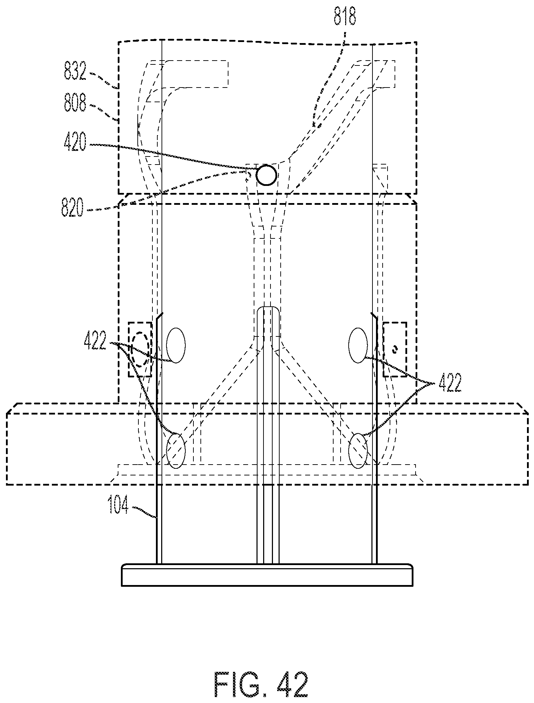

The disposable portion 104 is advanced further proximally to arrive in a third configuration, as shown in FIG. 42. In the third configuration, the protrusions 420 of the disposable portion 104 (one protrusion 420 being visible in FIG. 42) have moved to the distal portions 820 of the securing tracks 818 of the proximal cylinder 808 (one entire securing track 818 being visible in FIG. 42). The magnetic elements 422 of the disposable portion 104 (four magnetic elements 422 being visible in FIG. 42) may also magnetically couple with one or more magnetic elements of the reusable portion 102 to hold the disposable portion 104 in the third configuration.

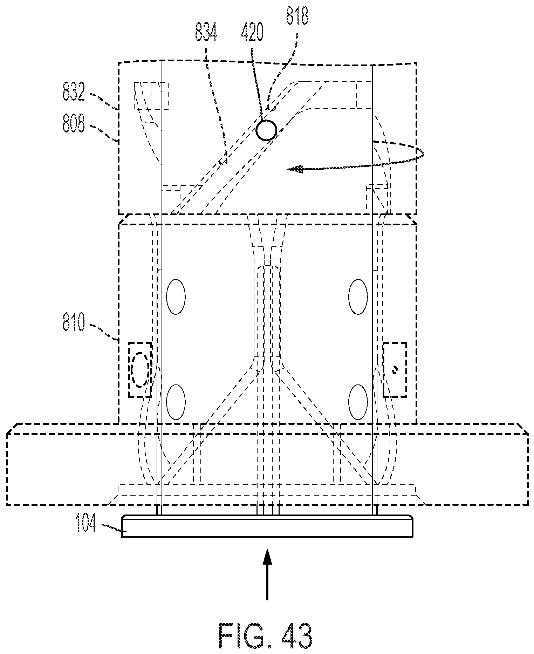

As shown in FIG. 43, the proximal cylinder 808 rotates relative to the distal cylinder 810 (illustratively, in a clockwise direction as viewed from the proximal end 108 of the device 100 - shown elsewhere) such that the securing mechanism 832 moves to a fourth configuration. In the fourth configuration, the protrusions 420 of the disposable portion 104 (one protrusion 420 being visible in FIG. 42) are disposed in the entry/egress portions 834 of the securing tracks 818 of the proximal cylinder 808 (one entire securing track 818 being visible in FIG. 43). As a result, the proximal cylinder 808 has pulled the disposable portion 104 proximally further into the securing mechanism 832.

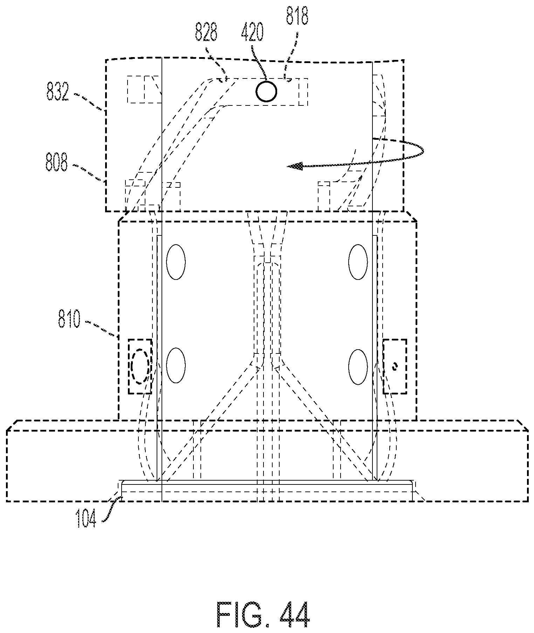

As shown in FIG. 44, the proximal cylinder 808 further rotates relative to the distal cylinder 810 (in the same direction - illustratively, in the clockwise direction as viewed from the proximal end 108) such that the securing mechanism 832 moves to a fifth configuration. In the fifth configuration, the protrusions 420 of the disposable portion 104 (one protrusion 420 being visible in FIG. 44) are disposed in the securing portions 828 of the securing tracks 818 of the proximal cylinder 808 (one securing portion 828 being visible in FIG. 44). This inhibits detachment of the disposable portion 104 from the reusable portion 102, and the securing mechanism 832 may remain in the fifth configuration until the therapeutic agent 106 is delivered from the syringe assembly 118 (both shown in FIG. 3).

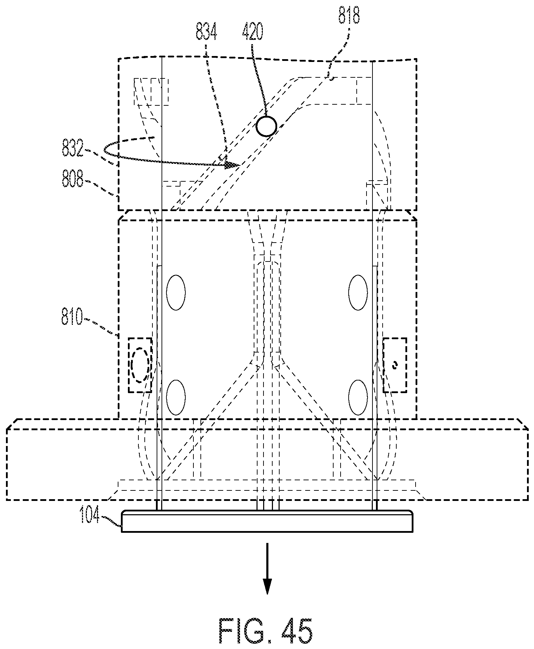

As shown in FIG. 45, the proximal cylinder 808 then rotates relative to the distal cylinder 810 (in the opposite direction - illustratively, in the counterclockwise direction as viewed from the proximal end 108) to arrive in a sixth configuration. In the sixth configuration, the protrusions 420 of the disposable portion 104 (one protrusion 420 being visible in FIG. 45) are disposed in the entry/egress portions 834 of the securing tracks 818 of the proximal cylinder 808 (one entire securing track 818 being visible in FIG. 45). As a result, the proximal cylinder 808 has begun pushing the protrusions 420 and the disposable portion 104 distally to eject the disposable portion 104.

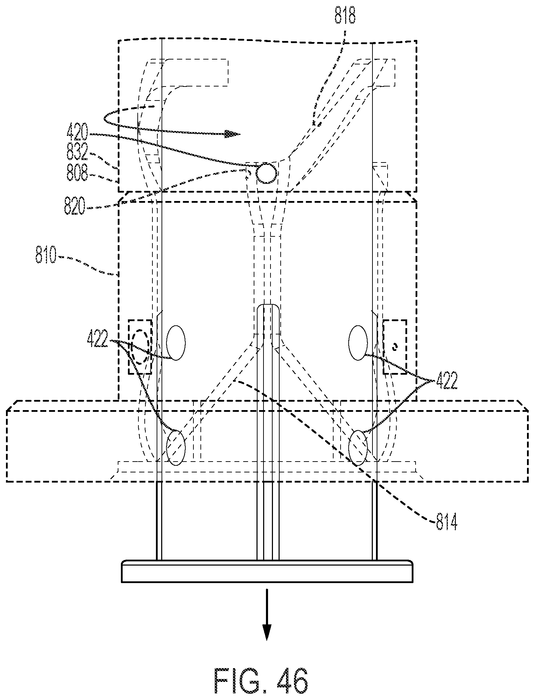

As shown in FIG. 46, the proximal cylinder 808 further rotates relative to the distal cylinder 810 (in the same direction - illustratively, in the counterclockwise direction as viewed from the proximal end 108) to arrive in a seventh configuration. In the seventh configuration, the protrusions 420 of the disposable portion 104 (one protrusion 420 being visible in FIG. 46) are disposed in the distal portions 820 of the securing tracks 818 of the proximal cylinder 808 (one entire securing track 818 being visible in FIG. 46). In the seventh configuration, the distal portions 820 of the securing tracks 818 are aligned with the leading tracks 814 of the distal cylinder 810 (one entire leading track 814 being visible in FIG. 46). The magnetic elements 422 may also magnetically couple with one or more magnetic elements of the reusable portion 102 hold the disposable portion 104 in the seventh configuration, although a user may pull the disposable portion 104 distally to detach the disposable portion 104 from the securing mechanism 832 and the reusable portion 102.

The electronics assembly 134 (FIG. 5) may cause the securing mechanism 832 and the disposable portion 104 to transition through the above configurations upon detecting one or more conditions in similar manners as described above in connection with the securing mechanism 132.

Although aspects of the disclosure mention a reusable portion and a disposable portion, any aspects disclosed herein could be adopted into a single disposable device. In this example, the first housing and the second housing would be referred to as a housing.

## Claims

1. A therapeutic agent delivery device (100), comprising:
a disposable portion (104, 504, 604), comprising:
a first housing (400, 500, 600) comprising a distal end (404);
a syringe assembly (118) carried by the first housing, the syringe assembly comprising:
a chamber (186) comprising a passageway (130);
a needle (120) in communication with the passageway;
the syringe assembly being translatable relative to the first housing from a stowed configuration to a deployed configuration, in the stowed configuration the needle being disposed proximally relative to the distal end of the first housing, and in the deployed configuration the needle at least partially extending distally from the distal end of the first housing;
a reusable portion (102) detachably carrying the disposable portion, the reusable portion comprising:
a second housing (112);
a drive mechanism (116) carried by the second housing and coupled to the syringe assembly,
a user input (114), the user input carried by the second housing and operatively coupled to the drive mechanism, wherein in response to actuation of the user input the drive mechanism is configured to translate the syringe assembly relative to the first housing from the stowed configuration to the deployed configuration,
wherein the user input comprises a first configuration and a second configuration prior to actuation, in the first configuration the drive mechanism inhibiting actuation of the user input, and in the second configuration the drive mechanism permitting actuation of the user input,
wherein in the first configuration the user input (114) is in a distal position, and in the second configuration the user input (114) is in a position proximal to the distal position,
wherein the drive mechanism (116) includes a guide (160), and the user input includes one or more hook members (178) that engage the guide when the user input (114) is in the first configuration, and
wherein the drive mechanism (116) includes a follower (156) rotatable relative to the guide (160), and a portion of the follower engages the one or more hook members (178) of the user input (114) during rotation to release the one or more hook members from the guide, thereby allowing the user input to move to the second configuration.

2. The therapeutic agent delivery device (100) of claim 1, wherein the user input (114) is actuatable by a user to actuate a rotary actuator (148) of the drive mechanism (116).

3. The therapeutic agent delivery device (100) of claim 2, wherein in the first configuration the follower (156) of the drive mechanism (116) is configured to contact the user input (114) to inhibit actuation of the user input, and in the second configuration the follower is disposed apart from and permits actuation of the user input.

4. The therapeutic agent delivery device (100) of any one of claims 2-3, wherein the second housing (112) comprises a distal end, in the deployed configuration the needle (120) at least partially extending distally from the distal end of the second housing (112), the reusable portion (102) further comprising a sensor (140) configured to detect contact between the distal end of the second housing (112) and the skin of a subject, the reusable portion inhibiting actuation of the user input (114) when the sensor does not detect contact between the distal end of the second housing and the skin of the subject, and the reusable portion permitting actuation of the user input when the sensor detects contact between the distal end of the second housing and the skin of the subject.

5. The therapeutic agent delivery device (100) of any one of claims 2-4, wherein the disposable portion (104, 504, 604) further comprises a needle shield (414, 616) detachably coupled to the distal end (404) of the first housing (400, 500, 600) and obscuring the needle (120) in the stowed configuration, the reusable portion (102) further comprising a sensor (140) configured to detect the needle shield, the reusable portion inhibiting actuation of the user input (114) when the sensor detects that the needle shield is coupled to the distal end of the first housing, and the reusable portion permitting actuation of the user input when the sensor detects that the needle shield is detached from the distal end of the first housing.

6. The therapeutic agent delivery device (100) of any preceding claim, wherein when the one or more hook members (178) are released from the guide (160), a spring (184) provides a biasing force to proximally move the user input (114) to the second configuration.

7. The therapeutic agent delivery device (100) of any of claims 1-5, wherein the chamber (186) of the syringe assembly (118) contains a therapeutic agent.

8. A method of moving the user input (114) of the therapeutic agent delivery device (100) of claim 1, comprising the steps of:
providing the therapeutic agent delivery device with the user input in the first configuration;
detecting a presence of a needle shield (414, 616), wherein the needle shield is detachably coupled to the distal end (404) of the first housing (400, 500, 600) and obscuring the needle (120) in the stowed configuration, the therapeutic agent delivery device inhibiting actuation of the user input when a sensor (140) detects that the needle shield is coupled to the distal end of the first housing; and
actuating the user input to the second configuration when the sensor detects that the needle shield is detached from the distal end of the housing.

9. The method of claim 8, wherein the user input (114) is in a distal position when in the first configuration, and in a position proximal to the distal position when in the second configuration.

10. The method of any one of claims 8-9, further comprising:
detecting contact between a distal end of the second housing (112) and the skin of a subject, the therapeutic agent delivery device inhibiting actuation of the user input (114) when the sensor (140) does not detect contact between the distal end of the second housing and the skin of the subject, and the actuating step further comprises:
actuating the user input to the second configuration when the sensor detects that the needle shield (414, 616) is detached from the distal end (404) of the first housing (400, 500, 600) and when the sensor detects contact between the distal end of the second housing and the skin of the subject.

## Patentansprüche

1. Vorrichtung für die Abgabe therapeutischer Mittel (100), umfassend:
einen Einwegabschnitt (104, 504, 604), umfassend:
ein erstes Gehäuse (400, 500, 600), umfassend ein distales Ende (404);
eine Spritzenanordnung (118), die von dem ersten Gehäuse getragen wird, wobei die Spritzenanordnung umfasst:
eine Kammer (186), umfassend einen Durchgang (130);
eine Nadel (120), die in Kommunikation mit dem Durchgang steht;
wobei die Spritzenanordnung relativ zu dem ersten Gehäuse aus einer verstauten Konfiguration in eine ausgefahrene Konfiguration verschiebbar ist, wobei die Nadel in der verstauten Konfiguration proximal relativ zu dem distalen Ende des ersten Gehäuses angeordnet ist und sich die Nadel in der ausgefahrenen Konfiguration mindestens teilweise distal von dem distalen Ende des ersten Gehäuses erstreckt;
einen wiederverwendbaren Abschnitt (102), der den Einwegabschnitt lösbar trägt, wobei der wiederverwendbare Abschnitt umfasst:
ein zweites Gehäuse (112);
einen Antriebsmechanismus (116), der von dem zweiten Gehäuse getragen wird und an die Spritzenanordnung gekoppelt ist,
eine Benutzereingabe (114), wobei die Benutzereingabe von dem zweiten Gehäuse getragen wird und mit dem Antriebsmechanismus wirkverbunden ist, wobei als Reaktion auf eine Betätigung der Benutzereingabe der Antriebsmechanismus konfiguriert ist, um die Spritzenanordnung relativ zu dem ersten Gehäuse von der verstauten Konfiguration in die ausgefahrene Konfiguration zu verschieben,
wobei die Benutzereingabe eine erste Konfiguration und eine zweite Konfiguration vor der Betätigung umfasst, wobei in der ersten Konfiguration der Antriebsmechanismus die Betätigung der Benutzereingabe hemmt und in der zweiten Konfiguration der Antriebsmechanismus die Betätigung der Benutzereingabe zulässt,
wobei sich die Benutzereingabe (114) in der ersten Konfiguration in einer distalen Position befindet, und sich die Benutzereingabe (114) in der zweiten Konfiguration in einer Position proximal zu der distalen Position befindet, wobei der Antriebsmechanismus (116) eine Führung (160) einschließt, und die Benutzereingabe ein oder mehrere Hakenelemente (178) einschließt, die in die Führung eingreifen, wenn sich die Benutzereingabe (114) in der ersten Konfiguration befindet, und
wobei der Antriebsmechanismus (116) einen Mitnehmer (156) einschließt, der relativ zu der Führung (160) drehbar ist, und ein Abschnitt des Mitnehmers während der Drehung in das eine oder die mehreren Hakenelemente (178) der Benutzereingabe (114) eingreift, um das eine oder die mehreren Hakenelemente von der Führung zu lösen, wodurch es der Benutzereingabe ermöglicht wird, sich in die zweite Konfiguration zu bewegen.

2. Vorrichtung für die Abgabe therapeutischer Mittel (100) nach Anspruch 1, wobei die Benutzereingabe (114) von einem Benutzer betätigbar ist, um einen Drehaktor (148) des Antriebsmechanismus (116) zu betätigen.

3. Vorrichtung für die Abgabe therapeutischer Mittel (100) nach Anspruch 2, wobei in der ersten Konfiguration der Mitnehmer (156) des Antriebsmechanismus (116) konfiguriert ist, um die Benutzereingabe (114) zu kontaktieren, um eine Betätigung der Benutzereingabe zu hemmen, und in der zweiten Konfiguration der Mitnehmer beabstandet von der Benutzereingabe angeordnet ist und eine Betätigung der Benutzereingabe zulässt.

4. Vorrichtung für die Abgabe therapeutischer Mittel (100) nach einem der Ansprüche 2 bis 3, wobei das zweite Gehäuse (112) ein distales Ende umfasst, wobei sich die Nadel (120) in der ausgefahrenen Konfiguration mindestens teilweise distal von dem distalen Ende des zweiten Gehäuses (112) erstreckt, wobei der wiederverwendbare Abschnitt (102) ferner einen Sensor (140) umfasst, der konfiguriert ist, um einen Kontakt zwischen dem distalen Ende des zweiten Gehäuses (112) und der Haut eines Subjekts zu erkennen, wobei der wiederverwendbare Abschnitt eine Betätigung der Benutzereingabe (114) verhindert, wenn der Sensor keinen Kontakt zwischen dem distalen Ende des zweiten Gehäuses und der Haut des Subjekts erkennt, und der wiederverwendbare Abschnitt eine Betätigung der Benutzereingabe zulässt, wenn der Sensor einen Kontakt zwischen dem distalen Ende des zweiten Gehäuses und der Haut des Subjekts erkennt.

5. Vorrichtung für die Abgabe therapeutischer Mittel (100) nach einem der Ansprüche 2 bis 4, wobei der Einwegabschnitt (104, 504, 604) ferner einen Nadelschutz (414, 616) umfasst, der lösbar gekoppelt an das distale Ende (404) des ersten Gehäuses (400, 500, 600) ist und die Nadel (120) in der verstauten Konfiguration verdeckt, wobei der wiederverwendbare Abschnitt (102) ferner einen Sensor (140) umfasst, der konfiguriert ist, um den Nadelschutz zu erkennen, wobei der wiederverwendbare Abschnitt die Betätigung der Benutzereingabe (114) verhindert, wenn der Sensor erkennt, dass der Nadelschutz an das distale Ende des ersten Gehäuses gekoppelt ist, und der wiederverwendbare Abschnitt die Betätigung der Benutzereingabe zulässt, wenn der Sensor erkennt, dass der Nadelschutz von dem distalen Ende des ersten Gehäuses gelöst ist.

6. Vorrichtung für die Abgabe therapeutischer Mittel (100) nach einem der vorstehenden Ansprüche, wobei, wenn das eine oder die mehreren Hakenelemente (178) von der Führung (160) gelöst werden, eine Feder (184) eine Vorspannkraft bereitstellt, um die Benutzereingabe (114) proximal in die zweite Konfiguration zu bewegen.

7. Vorrichtung für die Abgabe therapeutischer Mittel (100) nach einem der Ansprüche 1 bis 5, wobei die Kammer (186) der Spritzenanordnung (118) ein therapeutisches Mittel enthält.

8. Verfahren zum Bewegen der Benutzereingabe (114) der Vorrichtung für die Abgabe therapeutischer Mittel (100) nach Anspruch 1, umfassend die Schritte:
Bereitstellen der Vorrichtung für die Abgabe therapeutischer Mittel für die Benutzereingabe in der ersten Konfiguration;
Erkennen eines Vorhandenseins eines Nadelschutzes (414, 616), wobei der Nadelschutz lösbar an das distale Ende (404) des ersten Gehäuses (400, 500, 600) gekoppelt ist und die Nadel (120) in der verstauten Konfiguration verdeckt, wobei die Vorrichtung für die Abgabe therapeutischer Mittel die Betätigung der Benutzereingabe verhindert, wenn ein Sensor (140) erkennt, dass der Nadelschutz an das distale Ende des ersten Gehäuses gekoppelt ist; und
Betätigen der Benutzereingabe in die zweite Konfiguration, wenn der Sensor erkennt, dass der Nadelschutz von dem distalen Ende des Gehäuses gelöst ist.

9. Verfahren nach Anspruch 8, wobei sich die Benutzereingabe (114) in einer distalen Position befindet, wenn sie in der ersten Konfiguration ist, und in einer Position proximal zu der distalen Position, wenn sie in der zweiten Konfiguration ist.

10. Verfahren nach einem der Ansprüche 8 bis 9, ferner umfassend:
Erkennen eines Kontakts zwischen einem distalen Ende des zweiten Gehäuses (112) und der Haut eines Subjekts, wobei die Vorrichtung für die Abgabe therapeutischer Mittel die Betätigung der Benutzereingabe (114) verhindert, wenn der Sensor (140) keinen Kontakt zwischen dem distalen Ende des zweiten Gehäuses und der Haut des Subjekts erkennt, und der Schritt des Betätigens ferner umfasst:
Betätigen der Benutzereingabe in die zweite Konfiguration, wenn der Sensor erkennt, dass der Nadelschutz (414, 616) von dem distalen Ende (404) des ersten Gehäuses (400, 500, 600) gelöst ist, und wenn der Sensor einen Kontakt zwischen dem distalen Ende des zweiten Gehäuses und der Haut des Subjekts erkennt.

## Revendications

1. Dispositif d'administration d'un agent thérapeutique (100), comprenant :
une partie jetable (104, 504, 604), comprenant :
un premier logement (400, 500, 600) comprenant une extrémité distale (404) ;
un ensemble seringue (118) porté par le premier logement, l'ensemble seringue comprenant :
une chambre (186) comprenant une voie de passage (130) ;
une aiguille (120) en communication avec la voie de passage ;
l'ensemble seringue étant mobile en translation par rapport au premier logement d'une configuration escamotée à une configuration déployée, dans la configuration escamotée l'aiguille étant disposée de manière proximale par rapport à l'extrémité distale du premier logement, et dans la configuration déployée l'aiguille s'étendant au moins partiellement de manière distale depuis l'extrémité distale du premier logement ;
une partie réutilisable (102) portant de manière amovible la partie jetable, la partie réutilisable comprenant :
un second logement (112) ;
un mécanisme d'entraînement (116) porté par le second logement et accouplé à l'ensemble seringue,
une entrée utilisateur (114), l'entrée utilisateur étant portée par le second logement et accouplée de manière fonctionnelle au mécanisme d'entraînement, dans lequel, en réponse à l'actionnement de l'entrée utilisateur, le mécanisme d'entraînement est configuré pour déplacer en translation l'ensemble seringue par rapport au premier logement de la configuration escamotée à la configuration déployée,
dans lequel l'entrée utilisateur comprend une première configuration et une seconde configuration avant l'actionnement, dans la première configuration le mécanisme d'entraînement empêchant l'actionnement de l'entrée utilisateur, et dans la seconde configuration le mécanisme d'entraînement permettant l'actionnement de l'entrée utilisateur,
dans lequel, dans la première configuration, l'entrée utilisateur (114) est dans une position distale, et dans la seconde configuration, l'entrée utilisateur (114) est dans une position proximale par rapport à la position distale, dans lequel le mécanisme d'entraînement (116) comporte un guide (160), et l'entrée utilisateur comporte un ou plusieurs éléments de crochet (178) qui viennent en prise avec le guide lorsque l'entrée utilisateur (114) est dans la première configuration, et
dans lequel le mécanisme d'entraînement (116) comporte un suiveur (156) rotatif par rapport au guide (160), et une partie du suiveur vient en prise avec le ou les éléments de crochet (178) de l'entrée utilisateur (114) pendant la rotation pour libérer le ou les éléments de crochet du guide, permettant ainsi à l'entrée utilisateur de se déplacer vers la seconde configuration.

2. Dispositif d'administration d'un agent thérapeutique (100) selon la revendication 1, dans lequel l'entrée utilisateur (114) peut être actionnée par un utilisateur pour actionner un actionneur rotatif (148) du mécanisme d'entraînement (116).

3. Dispositif d'administration d'un agent thérapeutique (100) selon la revendication 2, dans lequel, dans la première configuration, le suiveur (156) du mécanisme d'entraînement (116) est configuré pour venir en contact avec l'entrée utilisateur (114) pour empêcher l'actionnement de l'entrée utilisateur, et dans la seconde configuration, le suiveur est disposé à l'écart de l'entrée utilisateur et permet l'actionnement de l'entrée utilisateur.

4. Dispositif d'administration d'un agent thérapeutique (100) selon l'une quelconque des revendications 2 à 3, dans lequel le second logement (112) comprend une extrémité distale, dans la configuration déployée l'aiguille (120) s'étendant au moins partiellement de manière distale depuis l'extrémité distale du second logement (112), la partie réutilisable (102) comprenant en outre un capteur (140) configuré pour détecter un contact entre l'extrémité distale du second logement (112) et la peau d'un sujet, la partie réutilisable empêchant l'actionnement de l'entrée utilisateur (114) lorsque le capteur ne détecte pas de contact entre l'extrémité distale du second logement et la peau du sujet, et la partie réutilisable permettant l'actionnement de l'entrée utilisateur lorsque le capteur détecte un contact entre l'extrémité distale du second logement et la peau du sujet.

5. Dispositif d'administration d'un agent thérapeutique (100) selon l'une quelconque des revendications 2 à 4, dans lequel la partie jetable (104, 504, 604) comprend en outre un protège-aiguille (414, 616) accouplé de manière amovible à l'extrémité distale (404) du premier logement (400, 500, 600) et masquant l'aiguille (120) dans la configuration escamotée, la partie réutilisable (102) comprenant en outre un capteur (140) configuré pour détecter le protège-aiguille, la partie réutilisable empêchant l'actionnement de l'entrée utilisateur (114) lorsque le capteur détecte que le protège-aiguille est accouplé à l'extrémité distale du premier logement, et la partie réutilisable permettant l'actionnement de l'entrée utilisateur lorsque le capteur détecte que le protège-aiguille est détaché de l'extrémité distale du premier logement.

6. Dispositif d'administration d'un agent thérapeutique (100) selon l'une quelconque revendication précédente, dans lequel, lorsque le ou les éléments de crochet (178) sont libérés du guide (160), un ressort (184) fournit une force de sollicitation pour déplacer de manière proximale l'entrée utilisateur (114) vers la seconde configuration.

7. Dispositif d'administration d'un agent thérapeutique (100) selon l'une quelconque des revendications 1 à 5, dans lequel la chambre (186) de l'ensemble seringue (118) contient un agent thérapeutique.

8. Procédé de déplacement de l'entrée utilisateur (114) du dispositif d'administration d'un agent thérapeutique (100) selon la revendication 1, comprenant les étapes de :
fourniture du dispositif d'administration d'un agent thérapeutique avec l'entrée utilisateur dans la première configuration ;
détection d'une présence d'un protège-aiguille (414, 616), dans lequel le protège-aiguille est accouplé de manière amovible à l'extrémité distale (404) du premier logement (400, 500, 600) et masquant l'aiguille (120) dans la configuration escamotée, le dispositif d'administration d'un agent thérapeutique empêchant l'actionnement de l'entrée utilisateur lorsqu'un capteur (140) détecte que le protège-aiguille est accouplé à l'extrémité distale du premier logement ; et
actionnement de l'entrée utilisateur vers la seconde configuration lorsque le capteur détecte que le protège-aiguille est détaché de l'extrémité distale du logement.

9. Procédé selon la revendication 8, dans lequel l'entrée utilisateur (114) est dans une position distale lorsqu'elle est dans la première configuration, et dans une position proximale par rapport à la position distale lorsqu'elle est dans la seconde configuration.

10. Procédé selon l'une quelconque des revendications 8 à 9, comprenant en outre :
la détection d'un contact entre une extrémité distale du second logement (112) et la peau d'un sujet, le dispositif d'administration d'un agent thérapeutique empêchant l'actionnement de l'entrée utilisateur (114) lorsque le capteur (140) ne détecte pas de contact entre l'extrémité distale du second logement et la peau du sujet, et l'étape d'actionnement comprend en outre :
l'actionnement de l'entrée utilisateur vers la seconde configuration lorsque le capteur détecte que le protège-aiguille (414, 616) est détaché de l'extrémité distale (404) du premier logement (400, 500, 600) et lorsque le capteur détecte un contact entre l'extrémité distale du second logement et la peau du sujet.
